# EUROPEAN PATENT APPLICATION

(11) **EP 4 446 341 A1**
(43) Date of publication of application: **16.10.2024**
(21) Application number: 22903521.7
(22) Date of filing: 07.12.2022
(51) Int. Cl.: C07K 16/28, C07K 19/00, C07K 14/725, C12N 15/62, C12N 15/13, A61P 35/00, A61P 37/02, G01N 33/68

(54) **ANTIBODY BINDING TO BCMA AND USE THEREOF**

(30) Priority: 07.12.2021 CN 202111487287
(71) Applicant: Innovent Biologics (Suzhou) Co., Ltd., Suzhou, Jiangsu 215123 (CN)
(72) Inventor: WEI, Huafeng, Suzhou, Jiangsu 215123 (CN); XU, Dan, Suzhou, Jiangsu 215123 (CN); DAROWSKI, Diana Binia, Suzhou, Jiangsu 215123 (CN); PRINZ, Bianka, Suzhou, Jiangsu 215123 (CN); BOLAND, Nadthakarn, Suzhou, Jiangsu 215123 (CN); GEOGHEGAN, James, Suzhou, Jiangsu 215123 (CN)
(74) Representative: Sagittarius IP
(86) International application number: PCT/CN2022/137266
(87) International publication number: WO 2023/104100

(57) **Abstract**

Provided are an antibody having an improved affinity for specifically binding to BCMA, an antibody further comprising a P329G mutation, and a conjugate, a fusion, a bispecific antibody or a pharmaceutical composition comprising the antibody. In addition, further provided are a nucleic acid encoding the antibody, a host cell comprising the nucleic acid, and a method for preparing the antibody. The present invention also relates to the therapeutic and diagnostic use of the antibody binding to BCMA.

## Description

### FIELD OF THE INVENTION

The present invention relates to the field of antibody medicine. Particularly, the present invention relates to an antibody having an improved affinity for specifically binding to B-cell maturation antigen (BCMA), an antibody further comprising a P329G mutation, and a composition comprising the antibody. In addition, the present invention relates to a nucleic acid encoding the antibody, a host cell comprising the nucleic acid, and a method for preparing the antibody. The invention also relates to the therapeutic and diagnostic uses of such BCMA-binding antibodies.

### BACKGROUND

B-cell maturation antigen (BCMA, namely CD269, TNFRSF17) is a member of tumor necrosis factor receptor superfamily (TNFRSF). BCMA is a type III transmembrane protein. It has a cysteine rich domain (CRD) in the extracellular domain (ECD) that is characteristic of TNFR family members, and this domain forms a ligand binding motif. The ligands of BCMA include B cell activating factor (BAFF) and B cell proliferation inducing ligand (a proliferation-inducing ligand, APRIL), and B cell proliferation inducing ligand (APRIL) binds to BCMA with higher affinity to promote tumor cell proliferation.

BCMA is mainly expressed on the surface of mature B cells (i.e., plasma cells), but not in normal hematopoietic stem cells and non blood source tissues. BCMA signaling is indispensable for the survival of long-lived bone marrow plasma cells, but not necessary for the overall B cell homeostasis. BCMA on the membrane surface can be cleaved by γ-secretase and falls off. The produced soluble BCMA (sBCMA) may reduce the BCMA signal transduction from the membrane surface by blocking BAFF/APRII, ligand binding. BCMA is found to be overexpressed in multiple myeloma (MM) cells in preclinical models and human tumors, which upregulates classical and non classical NF-xB signaling, promotes the growth, survival and adhesion of MM cells, and induces osteoclast activation, angiogenesis, metastasis and immunosuppression, etc. BCMA expression has become an important marker for the diagnosis of MM. In addition, the level of sBCMA in serum of MM patients increases, which is positively proportional to the number of MM cells in bone marrow, and its concentration change is closely related to the prognosis and treatment response of MM patients.

Multiple myeloma, also known as plasmacytoma or Kähler's disease, is a refractory B cell line malignant tumor characterized by abnormal proliferation of plasma cells. Since BCMA is only expressed in plasma cells and not expressed in natural and memory B cells, BCMA has become a hot target for the treatment of B cell malignant tumors, especially multiple myeloma. New BCMA specific binding molecules are still needed in the field. The present invention meets the requirement by providing antibodies that bind BCMA with high target specificity and high affinity, especially antibodies that bind BCMA expressed on the surface of tumor cells.

### SUMMARY OF THE INVENTION

Through research, inventors of the present invention have developed a group of novel anti-BCMA antibodies having high binding affinity to BCMA. The antibody that specifically binds to BCMA or antigen-binding fragment thereof in the present invention has one or more of the following properties:
(1) binding to BCMA, such as human BCMA, cynomolgus monkey BCMA and mouse BCMA, with a high affinity, for example, the binding of the anti-BCMA antibody or antigen-binding fragment thereof to BCMA has K_{D} of about 10⁻⁹M to about 10⁻¹²M, as measured by ForteBio Kinetic Binding Assay;
(2) specifically binding to BCMA expressed on the cell surface;
(3) having ADCC cytotoxic killing effect on cells expressing BCMA;
(4) having ADCP killing effect on cells expressing BCMA;
(5) blocking, inhibiting the growth of cells expressing human BCMA (especially multiple myeloma cells), and/or killing said cells; and
(6) having an *in vivo* anti-tumor effect on tumors expressing BCMA, and having no significant toxic and side effects.

In a first aspect, the present invention provides an antibody that specifically binds to BCMA or antigen-binding fragment thereof, comprising
(a) three CDRs in the amino acid sequence of heavy chain variable region shown in SEQ ID NO: 27 and three CDRs in the amino acid sequence of light chain variable region shown in SEQ ID NO: 36; or variants with a single CDR or several CDRs not exceeding 2 or 1 amino acid change per CDR region within the six CDR regions;
(b) three CDRs in the amino acid sequence of heavy chain variable region shown in SEQ ID NO: 45 and three CDRs in the amino acid sequence of light chain variable region shown in SEQ ID NO: 54; or variants with a single CDR or several CDRs not exceeding 2 or 1 amino acid change per CDR region within the six CDR regions;
(c) three CDRs in the amino acid sequence of heavy chain variable region shown in SEQ ID NO: 81 and three CDRs in the amino acid sequence of light chain variable region shown in SEQ ID NO: 90; or variants with a single CDR or several CDRs not exceeding 2 or 1 amino acid change per CDR region within the six CDR regions; or
(b) three CDRs in the amino acid sequence of heavy chain variable region shown in SEQ ID NO: 99 and three CDRs in the amino acid sequence of light chain variable region shown in SEQ ID NO: 108; or variants with a single CDR or several CDRs not exceeding 2 or 1 amino acid change per CDR region within the six CDR regions;
   wherein the amino acid change is the addition, deletion or substitution of amino acid.

In some embodiments, the antibody that specifically binds to BCMA or antigen-binding fragment thereof in the present invention comprises a heavy chain variable region and a light chain variable region, wherein according to Kabat numbering
(a) the heavy chain variable region comprises HCDR1 shown in GSIVSSSYYWT (SEQ ID NO: 19), or a variant of the HCDR1 with no more than 2 amino acid changes or no more than 1 amino acid change; HCDR2 shown in SISIAGSTYYNPSLKS (SEQ ID NO: 20), or a variant of the HCDR2 with no more than 2 amino acid changes or no more than 1 amino acid change; HCDR3 shown in ARDRGDTILDV (SEQ ID NO: 21), or a variant of the HCDR3 with no more than 2 amino acid changes or no more than 1 amino acid change; the light chain variable region comprises LCDR1 shown in RASQSISRYLN (SEQ ID NO: 28), or a variant of the LCDR1 with no more than 2 amino acid changes or no more than 1 amino acid change; LCDR2 shown in AASSLQS (SEQ ID NO: 29), or a variant of the LCDR2 with no more than 2 amino acid changes or no more than 1 amino acid change; and LCDR3 shown in QQKYFDIT (SEQ ID NO: 30), or a variant of the LCDR3 with no more than 2 amino acid changes or no more than 1 amino acid change;
(b) the heavy chain variable region comprises HCDR1 shown in GSIVSSSYYWT (SEQ ID NO: 37), or a variant of the HCDR1 with no more than 2 amino acid changes or no more than 1 amino acid change; HCDR2 shown in SISIAGSTYYNPSLKS (SEQ ID NO: 38), or a variant of the HCDR2 with no more than 2 amino acid changes or no more than 1 amino acid change; HCDR3 shown in ARDRGDQILDV (SEQ ID NO: 39), or a variant of the HCDR3 with no more than 2 amino acid changes or no more than 1 amino acid change; the light chain variable region comprises LCDR1 shown in RASQSISRYLN (SEQ ID NO: 46), or a variant of the LCDR1 with no more than 2 amino acid changes or no more than 1 amino acid change; LCDR2 shown in AASSLQS (SEQ ID NO: 47), or a variant of the LCDR2 with no more than 2 amino acid changes or no more than 1 amino acid change; and LCDR3 shown in QQKYFDIT (SEQ ID NO: 48), or a variant of the LCDR3 with no more than 2 amino acid changes or no more than 1 amino acid change;
(c) the heavy chain variable region comprises HCDR1 shown in GTFSNDVIS(SEQ ID NO: 73), or a variant of the HCDR1 with no more than 2 amino acid changes or no more than 1 amino acid change; HCDR2 shown in VIIPIFGIANYAQKFQG(SEQ ID NO: 74), or a variant of the HCDR2 with no more than 2 amino acid changes or no more than 1 amino acid change; HCDR3 shown in ARGRGYYSSWLLDI(SEQ ID NO: 75), or a variant of the HCDR3 with no more than 2 amino acid changes or no more than 1 amino acid change; the light chain variable region comprises LCDR1 shown in QASQDITNYLN(SEQ ID NO: 82), or a variant of the LCDR1 with no more than 2 amino acid changes or no more than 1 amino acid change; LCDR2 shown in DASNLET(SEQ ID NO: 83), or a variant of the LCDR2 with no more than 2 amino acid changes or no more than 1 amino acid change; and LCDR3 shown in QQAFDLIT(SEQ ID NO: 84), or a variant of the LCDR3 with no more than 2 amino acid changes or no more than 1 amino acid change; or
(d) the heavy chain variable region comprises HCDR1 shown in GTFSNDVIS(SEQ ID NO: 91), or a variant of the HCDR1 with no more than 2 amino acid changes or no more than 1 amino acid change; HCDR2 shown in VIIPIFGIANYAQKFQG(SEQ ID NO: 92), OR A VARIANT OF THE HCDR2 with no more than 2 amino acid changes or no more than 1 amino acid change; HCDR3 shown in ARGRGYYSSWLHDI(SEQ ID NO: 93), or a variant of the HCDR3 with no more than 2 amino acid changes or no more than 1 amino acid change; the light chain variable region comprises LCDR1 shown in QASQDITNYLN(SEQ ID NO: 100), or a variant of the LCDR1 with no more than 2 amino acid changes or no more than 1 amino acid change; LCDR2 shown in DASNLET(SEQ ID NO: 101), or a variant of the LCDR2 with no more than 2 amino acid changes or no more than 1 amino acid change; and LCDR3 shown in QQAFDLIT(SEQ ID NO: 102), or a variant of the LCDR3 with no more than 2 amino acid changes or no more than 1 amino acid change;
wherein the amino acid change is the addition, deletion or substitution of amino acid.

In some embodiments, the antibody that specifically binds to BCMA or antigen-binding fragment thereof in the present invention comprises a heavy chain variable region and a light chain variable region, wherein
(a) the heavy chain variable region comprises HCDR1 shown in GSIVSSSYYWT (SEQ ID NO: 19); HCDR2 shown in SISIAGSTYYNPSLKS (SEQ ID NO: 20); and HCDR3 shown in ARDRGDTILDV (SEQ ID NO: 21); the light chain variable region comprises LCDR1 shown in RASQSISRYLN (SEQ ID NO: 28); LCDR2 shown in AASSLQS (SEQ ID NO: 29); and LCDR3 shown in QQKYFDIT (SEQ ID NO: 30);
(b) the heavy chain variable region comprises HCDR1 shown in GSIVSSSYYWT (SEQ ID NO: 37); HCDR2 shown in SISIAGSTYYNPSLKS (SEQ ID NO: 38); and HCDR3 shown in ARDRGDQILDV (SEQ ID NO: 39); the light chain variable region comprises LCDR1 shown in RASQSISRYLN (SEQ ID NO: 46); LCDR2 shown in AASSLQS (SEQ ID NO: 47); and LCDR3 shown in QQKYFDIT (SEQ ID NO: 48);
(c) the heavy chain variable region comprises HCDR1 shown in GTFSNDVIS(SEQ ID NO: 73); HCDR2 shown in VIIPIFGIANYAQKFQG(SEQ ID NO: 74); HCDR3 shown in ARGRGYYSSWLLDI(SEQ ID NO: 75); the light chain variable region comprises LCDR1 shown in QASQDITNYLN(SEQ ID NO: 82); LCDR2 shown in DASNLET(SEQ ID NO: 83); and LCDR3 shown in QQAFDLIT(SEQ ID NO: 84); or
(d) the heavy chain variable region comprises HCDR1 shown in GTFSNDVIS(SEQ ID NO: 91); HCDR2 shown in VIIPIFGIANYAQKFQG(SEQ ID NO: 92); HCDR3 shown in ARGRGYYSSWLHDI(SEQ ID NO: 93); the light chain variable region comprises LCDR1 shown in QASQDITNYLN(SEQ ID NO: 100); LCDR2 shown in DASNLET(SEQ ID NO: 101); and
LCDR3 shown in QQAFDLIT(SEQ ID NO: 102).

In some embodiments, the antibody that specifically binds to BCMA or antigen-binding fragment thereof in the present invention comprises a heavy chain variable region and a light chain variable region, wherein
(a) the heavy chain variable region comprises a sequence shown in SEQ ID NO: 27 or a sequence having at least 90%, 91%, 92%, 93%, 94%, 95%, 96%, 97%, 98% or 99% identity thereto, and the light chain variable region comprises a sequence shown in SEQ ID NO: 36 or a sequence having at least 90%, 91%, 92%, 93%, 94%, 95%, 96%, 97%, 98% or 99% identity thereto;
(b) the heavy chain variable region comprises a sequence shown in SEQ ID NO: 45 or a sequence having at least 90%, 91%, 92%, 93%, 94%, 95%, 96%, 97%, 98% or 99% identity thereto, and the light chain variable region comprises a sequence shown in SEQ ID NO: 54 or a sequence having at least 90%, 91%, 92%, 93%, 94%, 95%, 96%, 97%, 98% or 99% identity thereto;
(c) the heavy chain variable region comprises a sequence shown in SEQ ID NO: 81 or a sequence having at least 90%, 91%, 92%, 93%, 94%, 95%, 96%, 97%, 98% or 99% identity thereto, and the light chain variable region comprises a sequence shown in SEQ ID NO: 90 or a sequence having at least 90%, 91%, 92%, 93%, 94%, 95%, 96%, 97%, 98% or 99% identity thereto; or
(d) the heavy chain variable region comprises a sequence shown in SEQ ID NO: 99 or a sequence having at least 90%, 91%, 92%, 93%, 94%, 95%, 96%, 97%, 98% or 99% identity thereto, and the light chain variable region comprises a sequence shown in SEQ ID NO: 108 or a sequence having at least 90%, 91%, 92%, 93%, 94%, 95%, 96%, 97%, 98% or 99% identity thereto.

In some embodiments, the antibody that specifically binds to BCMA or antigen-binding fragment thereof in the present invention comprises a heavy chain variable region and a light chain variable region, wherein said antibody or antigen binding fragment comprises
(a) a heavy chain variable region shown in SEQ ID NO: 27 and a light chain variable region shown in SEQ ID NO: 36;
(b) a heavy chain variable region shown in SEQ ID NO: 45 and a light chain variable region shown in SEQ ID NO: 54;
(c) a heavy chain variable region shown in SEQ ID NO: 81 and a light chain variable region shown in SEQ ID NO: 90;
(d) a heavy chain variable region shown in SEQ ID NO: 99 and a light chain variable region shown in SEQ ID NO: 108.

In some embodiments, the antibody that specifically binds to BCMA or antigen-binding fragment thereof in the present invention is IgG1, IgG2, IgG3 or IgG4 antibody; optionally, it is IgG1 or IgG4 antibody; optionally, it is IgG1 antibody. In some embodiments, said antigen-binding fragment is Fab, Fab', F(ab')₂, Fv, single chain Fv, single chain Fab, or diabody.

In some embodiments, the antibody that specifically binds to BCMA or antigen-binding fragment thereof in the present invention further comprises a mutant Fc domain, wherein the amino acid at position P329 according to EU numbering is mutated to glycine (G), and Fcy receptor binding of the mutant Fc domain is reduced, compared with Fcy receptor binding of the Fc domain of the parent antibody that is not mutated. For example, the mutant Fc domain is a mutant Fc domain of an IgG1, IgG2, IgG3 or IgG4 antibody; preferably, the mutant Fc domain is a mutant Fc domain of an IgG1 or IgG4 antibody; more preferably, the mutant Fc domain is a mutant Fc domain of an IgG1 antibody.

For example, the antibody or antigen-binding fragment comprises a heavy chain constant region sequence shown in SEQ ID NO: 111, or a sequence having at least 90%, 91%, 92%, 93%, 94%, 95%, 96%, 97%, 98% or 99% identity thereto and having the amino acid at position P329 according to EU numbering mutated to G.

For example, the antibody or antigen-binding fragment comprises a heavy chain constant region sequence shown in SEQ ID NO: 111, or a sequence having at least 90%, 91%, 92%, 93%, 94%, 95%, 96%, 97%, 98% or 99% identity thereto and having the amino acid at position P329 according to EU numbering mutated to G; and a light chain constant region sequence shown in SEQ ID NO: 112, or a sequence having at least 90%, 91%, 92%, 93%, 94%, 95%, 96%, 97%, 98% or 99% identity thereto.

For example, the antibody or antigen-binding fragment comprises a heavy chain constant region sequence shown in SEQ ID NO: 111 and a light chain constant region sequence shown in SEQ ID NO: 112.

In a second aspect, the present invention provides a nucleic acid encoding the antibody in the first aspect of the invention, a vector comprising the nucleic acid encoding the antibody, a host cell comprising the nucleic acid molecule or the vector, and a method for preparing the antibody. The method comprises the following steps: under conditions suitable for expressing the nucleic acid encoding the antibody that specifically binds to BCMA molecule or antigen-binding fragment thereof according to the first aspect of the invention, cultivating and introducing a host cell comprising an expression vector comprising the nucleic acid encoding the antibody that specifically binds to BCMA molecule or antigen-binding fragment thereof according to the first aspect of the invention, isolating the antibody that specifically binds to BCMA molecule or antigen-binding fragment thereof. Optionally, the method further comprises recovering the antibody that specifically binds to BCMA molecule or antigen-binding fragment thereof from the host cell. Preferably, the host cell is prokaryotic or eukaryotic, more preferably selected from Escherichia coli cells, yeast cells, mammalian cells or other cells suitable for producing the antibody or antigen-binding fragment thereof, and most preferably, the host cell is HEK293 cell or CHO cell.

In a third aspect, the present invention relates to a conjugate, a fusion or a bispecific antibody comprising the anti-BCMA antibody or antigen-binding fragment thereof in the first aspect of the present invention.

In a fourth aspect, the present invention relates to a pharmaceutical composition comprising the anti-BCMA antibody or antigen-binding fragment thereof in the first aspect of the present invention, or a conjugate, a fusion or a bispecific antibody in the third aspect of the present invention, and optionally a pharmaceutical carrier.

In a fifth aspect, the present invention relates to a use of the anti-BCMA antibody or antigen-binding fragment thereof in the first aspect of the invention, the conjugate, the fusion or the bispecific antibody in the third aspect of the invention, or the pharmaceutical composition in the fourth aspect of the invention in the preparation of a medicament for preventing or treating B cell related diseases in a subject. For example, the B cell related diseases are selected from: B cell malignant tumors, plasma cell malignant tumors, autoimmune diseases, preferably selected from: multiple myeloma, non-Hodgkin's lymphoma, B cell proliferation with uncertain malignant potential, lymphomatoid granulomatosis, post-transplant lymphoproliferative diseases, immune regulatory diseases, rheumatoid arthritis, myasthenia gravis, idiopathic thrombocytopenic purpura, antiphospholipid syndrome, Chagas disease, Graves' disease, Wegener's granulomatosis, polyarteritis nodosa, Schegren syndrome, pemphigus vulgaris, scleroderma, multiple sclerosis, ANCA-associated vasculitis, Goodpasture's disease, Kawasaki disease, autoimmune hemolytic anemia, and rapidly progressive glomerulonephritis, heavy chain disease, primary or immune cell related amyloidosis, or monoclonal gammopathy of undetermined significance, preferably, the B cell related disease is B cell malignant tumor, more preferably, multiple myeloma (MM) or non-Hodgkin's lymphoma (NHL).

In a sixth aspect, the present invention provides a kit for detecting BCMA in a sample. The kit comprises the anti-BCMA antibody or antigen-binding fragment thereof in the first aspect of the invention, which is used to perform the following steps:
(a) contacting a sample with the anti-BCMA antibody or antigen-binding fragment thereof in the first aspect of the invention; and
(b) detecting the formation of the complex between the anti-BCMA antibody or antigen-binding fragment thereof and BCMA; optionally, the anti-BCMA antibody or antigen-binding fragment thereof is detectably labeled.

### BRIEF DESCRIPTION OF THE DRAWINGS

When read together with the following drawings, the preferred embodiments of the present invention described in detail below will be better understood. For the purpose of illustrating the invention, the currently preferred embodiments are shown in the drawings. However, it should be understood that the present invention is not limited to the precise arrangement and means of the embodiments shown in the drawings.
Figure 1 shows that ADI-38497 antibody only binds to H929 cells expressing BCMA antigen, but not to BCMA-KO-H929 cells with BCMA gene knockout.
Figure 2A shows a schematic diagram of a method for determining antibody affinity using surface plasmon resonance (SPR).
Figure 2B shows the representative affinity profiles of ADI-38497 PG antibody with a recombinant BCMA from human, cynomolgus monkey, mouse, rat and rabbit, as determined by SPR.
Figure 2C shows the binding ability of P329G BCMA antibody to CHO-GS cells stably expressing human, cynomolgus monkey and mouse BCMA.
Figure 2D shows the binding activity of P329G BCMA antibody to positive BCMA-expressing multiple myeloma cell lines MM.1s, RPMI8226, U266, H929, L363 and AMO1.
Figure 3A shows the ability of ADI-38497 WT antibody and ADI-38497 PG antibody to mediate ADCC killing.
Figure 3B shows the ability of ADI-38497 WT antibody and ADI-38497 PG antibody to mediate ADCP killing.
Figure 3C shows the ability of ADI-38497 PG antibody to mediate target cell lysis.
Figure 4A and Figure 4B show the pharmacokinetic experiment results of ADI-38497 PG antibody in mice.
Figure 5A shows the therapeutic effect of ADI-38497 PG antibody in immunodeficient tumor bearing mice inoculated subcutaneously with highly BCMA-expressing human H929 tumor cells.
Figure 5B shows the weight changes of mice treated with ADI-38497 PG antibody in immunodeficient tumor bearing mice inoculated subcutaneously with highly BCMA-expressing human H929 tumor cells.
Figure 6A shows the anti-tumor effects of different doses of ADI-38497 PG antibody in the immunodeficient tumor bearing mice inoculated with human H929-luc tumor cells via tail vein.
Figure 6B shows the body weight changes of mice treated with ADI-38497 PG antibody in immunodeficient tumor bearing mice inoculated with human H929-luc tumor cells via tail vein.
Figure 7A shows the therapeutic effect of ADI-38497 PG antibody in immunodeficient tumor bearing mice inoculated subcutaneously with human H929 tumor cells.
Figure 7B shows the weight changes of mice treated with ADI-38497 PG antibody in immunodeficient tumor bearing mice inoculated subcutaneously with human H929 tumor cells.
Figure 7C and Figure 7D show the results of mouse hematologic and blood biochemistric tests when ADI-38497 PG antibody was used to treat immunodeficient tumor bearing mice inoculated subcutaneously with human H929 tumor cells.

### DETAILED DESCRIPTION

Unless otherwise defined, all technical and scientific terms used herein have the same meanings as those commonly understood by those skilled in the art to which the invention belongs. All publications, patent applications, patents and other references referred to herein are incorporated by reference in their entirety. Furthermore, the materials, methods and examples described herein are illustrative only and are not intended to be restrictive. Other features, objects and advantages of the present invention will be apparent from the description and drawings and from the appended claims.

### I. Definitions

For the purpose of interpretation of the present description, the following definitions will be used, and where appropriate, terms used in the singular may also include the plural, and vice versa. It is to be understood that the terms used herein are only intended to describe specific embodiments, and are not intended to be restrictive.

The term "about", when used in conjunction with a numerical value, means a numerical value that covers a range having a lower limit of 5% less than the specified numerical value and an upper limit of 5% greater than the specified numerical value.

As used herein, the terms "and/or" mean any one of the options or two or more of the options.

In the context, when the terms "comprise" or "comprising" are used, unless otherwise specified, the situation consisting of the elements, integers, or steps described is also covered. For example, when referring to a antibody variable region "comprising" a specific sequence, it is also intended to cover the antibody variable region consisting of the specific sequence.

The terms "BCMA" and "B cell maturation antigen" are used interchangeably, and include variants, isotypes, species homologues of human BCMA, and analogues having at least one identical epitope with BCMA (e.g., human BCMA). BCMA proteins may also include fragments of BCMA, such as extracellular domains and extracellular domain fragments, such as fragments that retain the ability to bind to any antibody of the invention.

As used herein, the terms "BCMA antibody", "antibody against BCMA", "antibody specifically binding to BCMA", "antibody specifically targeting to BCMA", and "antibody specifically recognizing BCMA" can be interchangeably used, meaning antibodies that can specifically bind to B cell maturation antigen (BCMA).

The term "antibody" is used herein in the broadest sense herein, referring to proteins comprising antigen binding sites, comprising natural and artificial antibodies of various structures, including but not limited to monoclonal antibodies, polyclonal antibodies, multi-specific antibodies (such as bispecific antibodies), single chain antibodies, complete antibodies and antibody fragments. Preferably, the antibody of the present invention is a single domain antibody or a heavy chain antibody.

"Antibody fragment" or "antigen-binding fragment" is used interchangeably herein, referring to a molecule different from a complete antibody, which comprises a part of a complete antibody and binds the antigen to which the complete antibody binds. Examples of antibody fragments include, but are not limited to, Fab, Fab', F (ab')2, Fv, single chain Fv, single chain Fab, or diabody.

An antibody that shows the same or similar binding affinity and/or specificity as a reference antibody refers to an antibody that can have at least 50%, 60%, 70%, 80%, 90% or more of the binding affinity and/or specificity of the reference antibody. This can be determined by any method known in the art for determine binding affinity and/or specificity.

"Complementarity determining regions" or "CDR regions" or "CDRs" are regions in the variable domain of an antibody that are hypervariable in sequence and form structurally defined loops ("hypervariable loops") and/or contain the antigen-contacting residues ("antigen contacts"). CDRs are mainly responsible for binding to an antigen epitope. Heavy chain CDRs are usually called CDR1, CDR2 and CDR3, and they are numbered sequentially from the N-terminus. In a given heavy chain variable region amino acid sequence, the exact amino acid sequence boundary of each CDR can be determined using any one or a combination of many well-known antibody CDR assignment systems, which include, for example, definitions of Chothia (Chothia et al. (1989) Nature 342: 877-883, Al-Lazikani et al. "Standard conformations for the canonical structures of immunoglobulins", Journal of Molecular Biology, 273, 927-948 (1997)) based on the three-dimensional structure and topology of CDR loops of an antibody, Kabat (Kabat et al., Sequences of Proteins of Immunological Interest, 4th edition, U.S. Department of Health and Human Services, National Institutes of Health (1987)), AbM (University of Bath), Contact (University College London), International ImMunoGeneTics database(IMGT) (http://imgt.cines.fr/) based on antibody sequence variability, and North CDR based on the affinity propagation clustering using a large number of crystal structures.

Unless otherwise stated, in the present invention, the term "CDRs" or "CDR sequences" cover CDR sequences determined in any of the above ways.

CDRs can also be determined based on having the same Kabat numbering positions as the reference CDR sequences, such as any of exemplified CDRs of the present invention. In the present invention, when referring to antibody variable region and specific CDR sequence (including heavy chain variable region residues), it refers to the numbering position according to Kabat numbering system.

Although CDRs are different between antibody and antibody, only a limited number of amino acid positions in CDRs directly participate in antigen binding. At least two of the Kabat, Chothia, AbM and Contact methods can be used to determine the minimum overlap region, thus providing a "minimum binding unit" for antigen binding. The minimum binding unit can be a sub part of a CDR. As is clear to those skilled in the art, residues in the rest of the CDR sequence can be determined through the structure and protein folding of the antibody. Therefore, the present invention also contemplates any variant of a CDR given herein. For example, in the variants of a CDR, the amino acid residues of the minimum binding unit can remain unchanged, while the remaining CDR residues defined by Kabat or Chothia or AbM can be replaced by conservative amino acid residues.

"Humanized" antibodies are chimeric antibodies comprising amino acid residues from non-human CDRs and amino acid residues from human FRs. In some embodiments, all or substantially all CDRs (e.g., CDRs) in humanized antibodies correspond to those of non-human antibodies, and all or substantially all FRs correspond to those of human antibodies. The humanized antibody may optionally comprise at least a portion of an antibody constant region derived from a human antibody. The "humanized version" of an antibody (such as, of a non-human antibody) refers to an antibody that has been humanized.

"Human antibody" means an antibody having an amino acid sequence corresponding to an amino acid sequence of an antibody generated by human or human cells, or of an antibody produced from a non-human source but using a human antibody library or other encoding sequences of a human antibody. Such a definition of a human antibody explicitly excludes a humanized antibody comprising non-human antigen-binding residues.

The term "Fc region" is used herein to define the C-terminal region of an immunoglobulin heavy chain, which comprises at least a portion of a constant region. The term includes natural sequence Fc region and variant Fc region. In some embodiments, the Fc region of a human IgG heavy chain extends from Cys226 or Pro230 to the carbonyl terminal of the heavy chain. However, the C-terminal lysine (Lys447) of Fc region may or may not exist. Unless otherwise specified, the numbering of amino acid residues in Fc region or constant region is according to EU numbering system, which is also called EU index, as described in Kabat et al., Sequences of Proteins of Immunological Interest, 5th Ed. Public Health Service, National Institutes of Health, Bethesda, MD, 1991.

In some embodiments, the Fc region of an immunoglobulin comprises two constant domains, namely CH2 and CH3, and in other embodiments, the Fc region of an immunoglobulin comprises three constant domains, namely CH2, CH3 and CH4.

The binding of IgG to Fcy receptor or C1q depends on the residues located in the hinge region and the CH2 domain. Two regions in the CH2 domain are critical to the binding to FcyR and complement C1q, and have a unique sequence in IgG2 and IgG4. It has been shown that the replacements of residues at positions 233-236 in human IgG1 and IgG2 and of residues at positions 327, 330 and 331 in human IgG4 can substantially reduce ADCC and CDC activities (Armour et al., Eur. J. Immunol. 29(8), 1999, 2613-2624; Shields et al., J. Biol. Chem 276(9), 2001, 6591-6604).

The terms "functional Fc region", "Fc region having function" and other similar terms are interchangable, and refer to Fc region with the effector function of a wild type Fc region.

Similar terms such as "variant Fc region", "Fc mutant", "mutation carrying Fc region", "mutant Fc region", "Fc region variant", "Fc variant", "variant Fc" and "mutant Fc" can be used interchangeably, referring to an Fc region comprising at least one amino acid modification and is different from the natural sequence Fc region/wild type Fc region.

In some embodiments, the variant Fc region comprises an amino acid sequence that differs from the amino acid sequence of the natural sequence Fc region by one or more amino acid substitutions, deletions, or additions. In some embodiments, the variant Fc region has at least one amino acid substitution compared with the Fc region of wild-type IgG, and the at least one amino acid substitution is to replace the amino acid at position P329 according to EU numbering with glycine (G).

"Fc receptor" or "FcR" means a molecule that binds to the Fc region of an antibody. In some embodiments, FcR is a natural human FcR. In some embodiments, FcR is a receptor that binds to IgG antibody, namely FcγR, including three receptors of FcγRI (CD64), FcγRII (CD32) and FcγRIII (CD16), as well as their allelic variants and alternative splicing forms. FcyRII receptors include FcγRIIA and FcγRIIB, and FcγRIII receptors include FcγRIIIA and FcγRIIIB_{∘}

The term "effector function" refers to those biological activities attributed to the Fc region of an immunoglobulin, which vary with immunoglobulin isotypes. Examples of immunoglobulin effector functions include Fc receptor binding, antibody dependent cell-mediated cytotoxicity (ADCC), antibody dependent cell phagocytosis (ADCP), cytokine secretion, immune complex mediated antigen presenting cell antigen-uptake, C1q binding, and complement dependent cytotoxicity (CDC), downregulation of cell surface receptors (such as B cell receptors), and B cell activation.

The term "antibody dependent cell-mediated cytotoxicity (ADCC)" is one of the main mechanisms by which certain cytotoxic effector cells (such as natural killer (NK) cells) mediate the killing of target cells and foreign host cells. In some embodiments, the antibody of the present invention provides antibody dependent cytotoxicity of T lymphocytes, and enhances antibody dependent cytotoxicity of NK cells.

The term "antibody dependent cell phagocytosis (ADCP)" refers to a cellular reaction, in which macrophages are induced to be activated by the binding of target-cell-binding-antibody to FcyRIIIa on the surface of macrophages, so as to internalize target cells then acidify and degrade in phagosomes. ADCP can also be mediated by FcyRIIa and FcyRI, but the proportion is relatively low.

The term "complement dependent cytotoxicity (CDC)" refers to the lysis of target cells in the presence of complements. The complement system is a part of the innate immune system, which is made up of a series of proteins. The proteins of the complement system are called "complements", which are represented by the abbreviation symbols C1, C2, C3, etc. They are a group of heat labile proteins that exist in human or vertebrate serum and tissue fluid and have enzymatic activity after activation. C1q is the first component of complement dependent cytotoxicity (CDC) pathway. It can bind six antibodies, but binding with two IgGs is sufficient to activate the complement cascade. The activation of the classical complement pathway is initiated by the binding of the first component (C1q) of the complement system to an antibody (of a suitable subclass) that binds to a related antigen, then a series of complement cascade reactions are activated, forming holes in the target cell membrane, thus leading to target cell death. To evaluate complement activation, a CDC assay can be performed according to a method described in, for example, Gazzano-Santoro et al., J. Immunol. Methods 202:163 (1996).

The term "variable region" or "variable domain" refers to a domain of antibody heavy chain or light chain involved in the binding of antibody to antigen. The variable domains of the heavy and light chains of natural antibodies usually have similar structures, and each domain comprises four conservative framework regions (FR) and three complementarity determining regions (CDR). (See, for example, Kindt et al., Kuby Immunology, 6th ed., W. H. Freeman and Co., page 91 (2007)). A single VH or VL domain may be sufficient to give antigen binding specificity.

As used herein, the terms "binding" or "specific binding" mean that the binding to antigen is selective and can be distinguished from unwanted or nonspecific interactions. The binding ability of an antibody to a specific antigen can be determined by enzyme-linked immunosorbent assay (ELISA), SPR or biolayer interferometry technology or other conventional binding assays known in the art.

"Conjugate" is an antibody conjugated with one or more other substances, including but not limited to cytotoxic agents or labels.

The term "inhibiting" or "blocking" refers to reducing certain parameters (e.g., activities) of a given molecule. For example, the term includes substances that cause the given molecule (e.g., BCMA) to be inhibited at least 5%, 10%, 20%, 30%, 40% or more of an activity. Therefore, the inhibition is not necessary to be 100%.

The terms "individual" or "subject" are used interchangeably, including mammals. Mammals include, but are not limited to, domesticated animals (e.g., cattle, sheep, cats, dogs, and horses), primates (e.g., human and non-human primates such as monkeys), rabbits, and rodents (e.g., mice and rats). In particular, the individual or subject is a human.

The terms "tumor" and "cancer" are used interchangeably herein, including solid tumors and liquid tumors.

The terms "cancer" and "cancerous" refer to physiological diseases in mammals with the cell growth uncontrolled.

The term "tumor" refers to the growth and proliferation of all neoplastic cells, whether malignant or benign, and all pre-cancerous and cancerous cells and tissues. The terms "cancer", "cancerous" and "tumor" are not mutually exclusive when mentioned herein.

As used herein, the term "label" refers to a compound or a composition that is directly or indirectly conjugated or fused to a reagent (such as an antibody) and promotes the detection of the reagent to which it is conjugated or fused. The label itself may be detectable (e.g., radioisotope label or fluorescent label) or, in the case of enzymatic labeling, the label may catalyze chemical changes in the substrate compound or composition to be detectable. The term is intended to cover the direct labeling of a probe or antibody by coupling (i.e., physically connecting) a detectable substance to a probe or antibody, and the indirect labeling of a probe or antibody by reacting with another reagent that is directly labeled. Examples of indirect labeling include the detection of a first antibody using a fluorescent labeled secondary antibody, and the end labeling of a DNA probe with biotin so that it can be detected using a fluorescent labeled streptavidin.

"Isolated" antibodies are those that have been separated from components of their natural environment. In some embodiments, BCMA antibodies are purified to more than 95% or 99% purity, as determined by, for example, electrophoresis (e.g., SDS-PAGE, isoelectric focusing (IEF), capillary electrophoresis) or chromatography (e.g., ion exchange or reverse phase HPLC). For a review of the methods used to assess antibody purity, see, for example, Flatman et al., J. Chromatogr. B848: 79-87 (2007).

"Isolated" nucleic acids refer to such nucleic acid molecules that have been separated from the components of their natural environment. The isolated nucleic acid includes a nucleic acid molecule contained in a cell normally containing the nucleic acid molecule, but the nucleic acid molecule exists outside a chromosome, or at a chromosome location different from its natural chromosome location. "Isolated nucleic acid encoding BCMA antibody" refers to one or more nucleic acid molecules encoding chains or segments of an BCMA antibody, including such a nucleic acid molecule in a single vector or separated vectors, and such a nucleic acid molecule in one or more locations in the host cell.

The sequence identity between sequences is calculated as follows.

In order to determine the identity percentage of two amino acid sequences or two nucleic acid sequences, the sequences are aligned for the purpose of optimal comparison (for example, gaps can be introduced in one or both of the first and second amino acid sequences or nucleic acid sequences for optimal comparison, or non homologous sequences can be discarded for the purpose of comparison). In one preferred embodiment, for comparison purposes, the length of the reference sequence in the alignment is at least 30%, preferably at least 40%, more preferably at least 50%, 60%, and even more preferably at least 70%, 80%, 90%, 100% of the reference sequence length. Then compare the amino acid residues or nucleotides at the corresponding amino acid position or nucleotide position. When the position in the first sequence is occupied by the same amino acid residues or nucleotides at the corresponding position in the second sequence, the molecules are the same at this position.

A mathematical algorithm can be used to compare two sequences and calculate percent identity between the sequences. In one preferred embodiment, the percent identity between two amino acid sequences is determined with the Needlema and Wunsch algorithm ((1970) J. Mol. Biol., 48: 444-453; available at http://www.gcg.com) which has been integrated into the GAP program of the GCG software package, using the Blossum 62 matrix or PAM250 matrix and a gap weight of 16, 14, 12, 10, 8, 6, or 4 and a length weight of 1, 2, 3, 4, 5, or 6. In another preferred embodiment, the percent identity between two nucleotide acid sequences is determined with the GAP program of the GCG software package (available at http://www.gcg.com), using the NWSgapdna.CMP matrix and a gap weight of 40, 50, 60, 70, or 80 and a length weight of 1, 2, 3, 4, 5, or 6. A particularly preferred parameter set (and one that should be used unless otherwise stated) is a Blossum 62 scoring matrix with a gap penalty of 12, a gap extension penalty of 4, and a frameshift gap penalty of 5.

The percent identity between two amino acid sequences or nucleotide sequences can also be determined with a PAM120 weighted remainder table, a gap length penalty of 12, and a gap penalty of 4, using the E. Meyers and W. Miller algorithm ((1989) CABIOS, 4: 11-17) which has been incorporated into the ALIGN program (version 2.0).

Additionally or alternatively, the nucleic acid sequences and protein sequences described herein can be further used as "query sequences" to perform searches against public databases to, e.g., identify other family member sequences or related sequences.

The terms "amino acid change" and "amino acid modification" are used interchangeably and refer to the addition, deletion, substitution and other modifications of amino acids. Any combination of amino acid addition, deletion, substitution and other modifications can be carried out, provided that the final polypeptide sequence has desired characteristics. In some embodiments, an amino acid substitution in the antibody results in reduced binding of the antibody to Fc receptor. For the purpose of changing for example the binding characteristic to the Fc region, it is particularly preferred to perform a non-conservative amino acid substitution, that is, to replace an amino acid with another amino acid having different structural and/or chemical properties. Amino acid substitution includes substitution with non naturally occurring amino acids or derivatives of naturally occurring twenty standard amino acids (e.g., 4-hydroxyproline, 3-methylhistidine, ornithine, homoserine, 5-hydroxylysine). Genetic or chemical methods known in the art can be used to generate amino acid changes. Genetic methods can include site directed mutagenesis, PCR, gene synthesis, etc. It may be useful to change the side chain groups of amino acids by methods other than genetic engineering, such as chemical modification. In the context, several names can be used to represent the same amino acid change. For example, the substitution of proline at position 329 of Fc domain to glycine can be expressed as 329G, G329, G₃₂₉, P329G, Pro329Gly, or "PG" for short.

The terms "conservative sequence modification" and "conservative sequence change" refer to amino acid modification or change that does not significantly affect or change the binding characteristic of antibodies or antibody fragments comprising amino acid sequences. Such conservative modifications include amino acid substitution, addition and deletion. The antibody or antibody fragment of the invention can be modified by standard techniques known in the art, such as site directed mutagenesis and PCR mediated mutagenesis. Conservative substitution is the substitution of an amino acid residue by an amino acid residue having a similar side chain. The families of amino acid residues with similar side chains have been defined in the art. These families include amino acids with basic side chains (e.g., lysine, arginine, histidine), acidic side chains (e.g., aspartic acid, glutamic acid), non charged polar side chains (e.g., glycine, asparagine, glutamine, serine, threonine, tyrosine, cysteine, tryptophan), non-polar side chains (e.g., alanine, valine, leucine, isoleucine, proline, phenylalanine, methionine), β-side chains (e.g. threonine, valine, isoleucine) and aromatic side chains (e.g. tyrosine, phenylalanine, tryptophan, histidine).

The term "pharmaceutical composition" refers to a composition that exists in a form that allows the biological activity of the active ingredient comprised therein to be effective, and does not comprise other ingredients that have unacceptable toxicity to the subject administrating the composition.

The term "pharmaceutically acceptable carrier" refers to diluents, adjuvants (such as (complete and incomplete) Freund's adjuvant), excipients, buffers or stabilizers administered together with active substances.

The term "BCMA associated diseases" refers to any disease induced, aggravated or otherwise associated with increased expression or activity of BCMA.

When used herein, "treatment" refers to slowing down, interrupting, blocking, relieving, stopping, reducing, or reversing the progress or severity of existing symptoms, disorders, conditions, or diseases. Desired therapeutic effects include, but are not limited to, preventing the occurrence or recurrence of the disease, alleviating symptoms, reducing any direct or indirect pathological consequences of the disease, preventing metastasis, reducing the rate of disease progression, improving or mitigating disease status, and relieving or improving prognosis. In some embodiments, the antibody molecules of the present invention are used to delay the development of a disease or to slow down the progress of a disease.

As used herein, "prevention" includes inhibition of the occurrence or progress of a disease or disorder or a symptom of a particular disease or disorder. In some embodiments, a subject with a family history of cancer is a candidate for a preventive protocol. In general, in the context of cancer, the term "prevention" refers to the administration of drugs before the onset of symptoms or signs of cancer, especially in a subject at risk of cancer.

The term "effective amount" refers to an amount or dosage of the antibody or composition of the present invention which generates expected effects in a patient in need of treatment or prevention after administration to the patient in a single dose or multiple doses. The effective amount can be easily determined by an attending physician as a person skilled in the art by considering a variety of factors as follows: such as mammal species, body weight, age, and general health condition; the specific disease involved; the extent or severity of the disease; response in an individual patient; specific antibody administered; mode of administration; bioavailability characteristics of the administered preparation; selected administration regimen; and use of any concomitant therapy.

"Therapeutically effective amount" refers to an amount effective to achieve a desired therapeutic result at a necessary dose for a necessary period of time. The therapeutically effective amount of an antibody or antibody fragments, or a composition thereof may vary depending on a variety of factors such as disease state, age, sex, and body weight of an individual, and the ability of the antibody or antibody portion to elicit a desired response in an individual. The therapeutically effective amount is also such an amount that any toxic or adverse effect of the antibody or antibody fragment, or the composition thereof is inferior to the therapeutically beneficial effect. "Therapeutically effective amount" preferably inhibits a measurable parameter (e.g., tumor growth rate, tumor volume, etc.) by at least about 20%, more preferably at least about 40%, even more preferably at least about 50%, 60%, or 70%, and still more preferably at least about 80% or 90%, relative to untreated subjects. The ability of a compound to inhibit a measurable parameter (e.g., cancer) can be evaluated in an animal model system that predicts efficacy in human tumors.

"Prophylactically effective amount" refers to an amount effective to achieve a desired prophylactic result at a necessary dose for a necessary period of time. Generally, since a prophylactic dose is administered in a subject before or at an earlier stage of a disease, a prophylactically effective amount will be less than a therapeutically effective amount.

When referring to nucleic acids herein, the term "vector" as used refers to a nucleic acid molecule capable of proliferating another nucleic acid to which it is linked. The term includes a vector that serve as a self-replicating nucleic acid structure as well as a vector linked to the genome of a host cell into which the vector has been introduced. Some vectors are capable of guiding the expression of nucleic acids operatively linked thereto. Such vectors are referred to as "expression vectors" herein.

The term "host cell" refers to a cell into which an exogenous polynucleotide has been introduced, including progeny of such cells. Host cells include "transformants" and "transformed cells", which include primary transformed cells and progeny derived therefrom, regardless of the number of passages. Progeny may not be exactly the same as parent cells in terms of nucleic acid content, and may comprise mutations. Mutant progenies having the same function or biological activities that are screened or selected from the initially transformed cells are included herein. Host cells are any type of cell systems that can be used to produce the antibody molecule of the present invention, including eukaryotic cells, e.g., mammalian cells, insect cells, and yeast cells; and prokaryotic cells, e.g., *E. coli* cells. Host cells include cultured cells, as well as cells within a transgenic animal, a transgenic plant, or a cultured plant tissue or animal tissue.

"Subject/patient sample" refers to the collection of cells, tissues or body fluids obtained from patients or subjects. The source of tissue or cell samples can be solid tissue, such as fresh, frozen and/or preserved organ or tissue samples or biopsy samples or puncture samples; Blood or any blood component; body fluids, such as cerebrospinal fluid, amniotic fluid (amniotic water), peritoneal fluid (ascites), or interstitial fluid; cells from the subject at any time of pregnancy or development. Tissue samples may contain compounds that do not mix with tissues in nature, such as preservatives, anticoagulants, buffers, fixatives, nutrients, antibiotics, etc. Examples of tumor samples herein include but are not limited to tumor biopsy, fine needle aspirates, bronchial lavage fluid, pleural fluid (pleural effusion), sputum, urine, surgical specimens, circulating tumor cells, serum, plasma, circulating plasma proteins, ascites, primary cell cultures or cell lines derived from or exhibiting tumor like characteristics, and preserved tumor samples, such as formalin fixed, paraffin embedded tumor samples or frozen tumor samples.

### II. Antibody of the present invention that specifically binds to BCMA molecules and antibody comprising a mutant Fc domain

The present invention provides an antibody that binds to BCMA with high target specificity and high affinity, comprising a heavy chain variable region and a light chain variable region, wherein
(a) the heavy chain variable region comprises HCDR1 shown in GSIVSSSYYWT (SEQ ID NO: 19), or a variant of the HCDR1 with no more than 2 amino acid changes or no more than 1 amino acid change; HCDR2 shown in SISIAGSTYYNPSLKS (SEQ ID NO: 20), or a variant of the HCDR2 with no more than 2 amino acid changes or no more than 1 amino acid change; HCDR3 shown in ARDRGDTILDV (SEQ ID NO: 21), or a variant of the HCDR3 with no more than 2 amino acid changes or no more than 1 amino acid change; the light chain variable region comprises LCDR1 shown in RASQSISRYLN (SEQ ID NO: 28), or a variant of the LCDR1 with no more than 2 amino acid changes or no more than 1 amino acid change; LCDR2 shown in AASSLQS (SEQ ID NO: 29), or a variant of the LCDR2 with no more than 2 amino acid changes or no more than 1 amino acid change; and LCDR3 shown in QQKYFDIT (SEQ ID NO: 30), or a variant of the LCDR3 with no more than 2 amino acid changes or no more than 1 amino acid change;
(b) the heavy chain variable region comprises HCDR1 shown in GSIVSSSYYWT (SEQ ID NO: 37), or a variant of the HCDR1 with no more than 2 amino acid changes or no more than 1 amino acid change; HCDR2 shown in SISIAGSTYYNPSLKS (SEQ ID NO: 38), or a variant of the HCDR2 with no more than 2 amino acid changes or no more than 1 amino acid change; HCDR3 shown in ARDRGDQILDV (SEQ ID NO: 39), or a variant of the HCDR3 with no more than 2 amino acid changes or no more than 1 amino acid change; the light chain variable region comprises LCDR1 shown in RASQSISRYLN (SEQ ID NO: 46), or a variant of the LCDR1 with no more than 2 amino acid changes or no more than 1 amino acid change; LCDR2 shown in AASSLQS (SEQ ID NO: 47), or a variant of the LCDR2 with no more than 2 amino acid changes or no more than 1 amino acid change; and LCDR3 shown in QQKYFDIT (SEQ ID NO: 48), or a variant of the LCDR3 with no more than 2 amino acid changes or no more than 1 amino acid change;
(c) the heavy chain variable region comprises HCDR1 shown in GTFSNDVIS(SEQ ID NO: 73), or a variant of the HCDR1 with no more than 2 amino acid changes or no more than 1 amino acid change; HCDR2 shown in VIIPIFGIANYAQKFQG(SEQ ID NO: 74), or a variant of the HCDR2 with no more than 2 amino acid changes or no more than 1 amino acid change; HCDR3 shown in ARGRGYYSSWLLDI(SEQ ID NO: 75), or a variant of the HCDR3 with no more than 2 amino acid changes or no more than 1 amino acid change; the light chain variable region comprises LCDR1 shown in QASQDITNYLN(SEQ ID NO: 82), or a variant of the LCDR1 with no more than 2 amino acid changes or no more than 1 amino acid change; LCDR2 shown in DASNLET(SEQ ID NO: 83), or a variant of the LCDR2 with no more than 2 amino acid changes or no more than 1 amino acid change; and LCDR3 shown in QQAFDLIT(SEQ ID NO: 84), or a variant of the LCDR3 with no more than 2 amino acid changes or no more than 1 amino acid change; or
(d) the heavy chain variable region comprises HCDR1 shown in GTFSNDVIS(SEQ ID NO: 91), or a variant of the HCDR1 with no more than 2 amino acid changes or no more than 1 amino acid change; HCDR2 shown in VIIPIFGIANYAQKFQG(SEQ ID NO: 92), or a variant of the HCDR2 with no more than 2 amino acid changes or no more than 1 amino acid change; HCDR3 shown in ARGRGYYSSWLHDI(SEQ ID NO: 93), or a variant of the HCDR3 with no more than 2 amino acid changes or no more than 1 amino acid change; the light chain variable region comprises LCDR1 shown in QASQDITNYLN(SEQ ID NO: 100), or a variant of the LCDR1 with no more than 2 amino acid changes or no more than 1 amino acid change; LCDR2 shown in DASNLET(SEQ ID NO: 101), or a variant of the LCDR2 with no more than 2 amino acid changes or no more than 1 amino acid change; and LCDR3 shown in QQAFDLIT(SEQ ID NO: 102), or a variant of the LCDR3 with no more than 2 amino acid changes or no more than 1 amino acid change;
wherein the change of amino acid is the addition, deletion or substitution of amino acid.

In some embodiments, the BCMA molecule binding antibody of the present invention binds to BCMAs from mammals, such as human, cynomolgus monkey, mouse, rat and rabbit.

In some embodiments, the BCMA molecule binding antibody of the present invention comprises a heavy chain variable region and a light chain variable region that specifically bind to BCMA, wherein
(a) the heavy chain variable region comprises a sequence shown in SEQ ID NO: 27 or a sequence having at least 90%, 91%, 92%, 93%, 94%, 95%, 96%, 97%, 98% or 99% identity thereto, and the light chain variable region comprises a sequence shown in SEQ ID NO: 36 or a sequence having at least 90%, 91%, 92%, 93%, 94%, 95%, 96%, 97%, 98% or 99% identity thereto;
(b) the heavy chain variable region comprises a sequence shown in SEQ ID NO: 45 or a sequence having at least 90%, 91%, 92%, 93%, 94%, 95%, 96%, 97%, 98% or 99% identity thereto, and the light chain variable region comprises a sequence shown in SEQ ID NO: 54 or a sequence having at least 90%, 91%, 92%, 93%, 94%, 95%, 96%, 97%, 98% or 99% identity thereto;
(c) the heavy chain variable region comprises a sequence shown in SEQ ID NO: 81 or a sequence having at least 90%, 91%, 92%, 93%, 94%, 95%, 96%, 97%, 98% or 99% identity thereto, and the light chain variable region comprises a sequence shown in SEQ ID NO: 90 or a sequence having at least 90%, 91%, 92%, 93%, 94%, 95%, 96%, 97%, 98% or 99% identity thereto; or
(d) the heavy chain variable region comprises a sequence shown in SEQ ID NO: 99 or a sequence having at least 90%, 91%, 92%, 93%, 94%, 95%, 96%, 97%, 98% or 99% identity thereto, and the light chain variable region comprises a sequence shown in SEQ ID NO: 108 or a sequence having at least 90%, 91%, 92%, 93%, 94%, 95%, 96%, 97%, 98% or 99% identity thereto;
wherein the amino acid change in the sequence having at least 90%, 91%, 92%, 93%, 94%, 95%, 96%, 97%, 98% or 99% identity is preferably amino acid substitution, more preferably amino acid conservative substitution, and preferably, the amino acid change does not occur in the CDR regions.

In some embodiments, the BCMA molecule binding antibody of the present invention is IgG1, IgG2, IgG3 or IgG4 antibody; preferably, it is IgG1 or IgG4 antibody; more preferably, it is IgG1 antibody, for example, human IgG1 antibody.

In some embodiments, the BCMA molecule binding antibody provided in the present invention comprises a mutant Fc domain, wherein the amino acid at position P329 according to EU numbering is mutated to glycine (G), and Fcy receptor binding of the mutant Fc domain is reduced, compared with Fcy receptor binding of the Fc domain of the parent antibody that is not mutated. For example, the mutant Fc domain is a mutant Fc domain of an IgG1, IgG2, IgG3 or IgG4 antibody; preferably, the mutant Fc domain is a mutant Fc domain of an IgG1 or IgG4 antibody; more preferably, the mutant Fc domain is a mutant Fc domain of an IgG1 antibody. For example, the mutant Fc domain is a mutant Fc domain of a human IgG1 antibody.

BCMA molecule binding antibodies comprising P329G mutant Fc domain cannot exert antibody dependent cytotoxicity by binding to Fcy receptor, and cannot exert antibody dependent phagocytosis (ADCP).

In some embodiments, the antibody that binds to BCMA molecule according to the present invention has one or more of the following properties:
(1) binding to BCMA, such as human BCMA, cynomolgus monkey BCMA and mouse BCMA, with a high affinity, for example, the binding of the anti-BCMA antibody or antigen-binding fragment thereof to BCMA has K_{D} of about 10⁻⁹M to about 10⁻¹²M, as measured by ForteBio Kinetic Binding Assay;
(2) specifically binding to BCMA expressed on the cell surface;
(3) having ADCC cytotoxic killing effect on cells expressing BCMA;
(4) having ADCP killing effect on cells expressing BCMA;
(5) blocking, inhibiting the growth of cells expressing human BCMA (especially multiple myeloma cells), and/or killing said cells; and
(6) having an *in vivo* anti-tumor effect on tumors expressing BCMA, and having no significant toxic and side effects.

In some embodiments, the present invention provides a nucleic acid encoding any of the above antibody that binds to BCMA molecule, or fragment thereof, or any chain thereof. In one embodiment, a vector comprising the nucleic acid is provided. In one embodiment, the vector is an expression vector. In one embodiment, a host cell comprising the nucleic acid or the vector is provided. In one embodiment, the host cell is eukaryotic. In another embodiment, the host cell is selected from yeast cells, mammalian cells (such as CHO cells or HEK293 cells), or other cells suitable for preparing the antibody or antigen-binding fragment thereof. In another embodiment, the host cell is prokaryotic.

For example, the nucleic acids of the present invention comprise nucleic acids encoding the antibody of the present invention that binds to BCMA molecule. In some embodiments, one or more vectors comprising the nucleic acids are provided. In one embodiment, the vector is an expression vector, such as a eukaryotic expression vector. Vectors include but are not limited to viruses, plasmids, cosmids, λ phages or yeast artificial chromosomes (YAC). In one embodiment, the vector is a pcDNA3.4 expression vector.

Once the expression vector or DNA sequence has been prepared for expression, the expression vector can be transfected or introduced into suitable host cells. Various techniques can be used for this purpose, for example, protoplast fusion, calcium phosphate precipitation, electroporation, retroviral transduction, viral transfection, biolistics, lipid-based transfection, or other conventional techniques. In the case of protoplast fusion, cells are cultured in a medium and screened for appropriate activity. Methods and conditions for culturing the resulting transfected cells and for recovering the resulting antibody molecules are known to those skilled in the art and may be changed or optimized according to the particular expression vector and the particular mammalian host cell used based on the present description and methods known in the prior art.

Additionally, cells having stably incorporated DNA in chromosomes thereof can be selected by introducing one or more markers permitting the selection of transfected host cells. The markers may, for example, provide prototrophy, biocidal (e.g., antibiotics) resistance, or heavy metal (e.g., copper) resistance, etc., for an auxotrophic host. Selectable marker genes may be connected directly to a DNA sequence to be expressed or introduced through co-transformation into the same cell. Additional elements may also be required for optimal synthesis of mRNA. The elements may include splicing signals, transcriptional promoters, enhancers, and termination signals.

In one embodiment, provided is a host cell comprising the polynucleotide of the present invention. In some embodiments, provided is a host cell comprising the expression vector of the present invention. In some embodiments, the host cell is selected from a yeast cell, a mammalian cell, and other cells suitable for preparing an antibody. Suitable host cells include prokaryotic microorganisms, such as *E. coli.* The host cells may also be eukaryotic microorganisms such as filamentous fungi or yeast, or various eukaryotic cells such as insect cells. Vertebrate cells may also be used as hosts. For example, a mammalian cell line engineered to be suitable for suspension growth may be used. Examples of useful mammalian host cell lines include monkey kidney CV1 line (COS-7) transformed by SV40; human embryonic kidney line (HEK 293 or 293F cells), baby hamster kidney cell (BHK), monkey kidney cell (CV1), African green monkey kidney cell (VERO-76), human cervical cancer cell (HELA), canine kidney cell (MDCK), buffalo rat liver cell (BRL 3A), human lung cell (W138), human liver cell (Hep G2), Chinese hamster ovary cell (CHO cell), CHO-S cell, NSO cell, and myeloma cell line such as Y0, NS0, P3X63, and Sp2/0. For reviews of mammalian host cell lines suitable for protein production, see, e.g., Yazaki and Wu, Methods in Molecular Biology, vol. 248 (B. K. C. Lo ed., Humana Press, Totowa, NJ), pp. 255-268 (2003). In one preferred embodiment, the host cell is a CHO cell or a HEK293 cell.

In one embodiment, the present invention provides a method for preparing antibodies (including P329G mutant antibodies) that bind to BCMA molecule, which comprises culturing a host cell comprising a nucleic acid encoding the antibody (including P329G mutant antibody) that binds to BCMA molecule or an expression vector comprising the nucleic acid under conditions suitable for expressing the nucleic acid encoding the antibody (including P329G mutant antibody) that binds to BCMA molecule, and optionally isolating the antibody (including P329G mutant antibody) that binds to BCMA molecule. In a certain embodiment, the method further comprises recovering the antibody (including P329G mutant antibody) that binds to BCMA molecule from the host cell (or host cell culture medium).

The antibody (including P329G mutant antibody) that binds to BCMA molecule prepared as described herein can be purified by known prior art such as high performance liquid chromatography, ion exchange chromatography, gel electrophoresis, affinity chromatography, size exclusion chromatography, etc. The actual conditions used to purify a particular protein also depend on factors such as net charge, hydrophobicity, hydrophilicity, etc., and these will be apparent to those skilled in the art. The purity of the antibody (including P329G mutant antibody) of the present invention that binds to BCMA molecule can be determined by any of a variety of well-known analytical methods, which include size exclusion chromatography, gel electrophoresis, high performance liquid chromatography, and the like.

The antibody (including P329G mutant antibody) provided herein that binds to BCMA molecule can be identified, screened, or characterized for physical/chemical properties and/or biological activities through a variety of assays known in the art. In one aspect, the antibody (including P329G mutant antibody) of the invention that binds to BCMA molecule is tested for the antigen-binding activity, for example, by known methods such as FACS, ELISA or Western blotting. The binding to BCMA can be determined by methods known in the art, and exemplary methods are disclosed herein. In some embodiments, the binding of the BCMA molecule-binding antibody (including P329G mutant antibody) of the present invention to BCMA (such as human BCMA) on cell surface is determined by SPR or biolayer interferometry.

The present invention also provides an assay for identifying the antibody (including P329G mutant antibody) having biological activity that binds to BCMA molecule. The biological activity can include, for example, ADCC effect, CDC effect, etc.

Cells used in any of the above *in vitro* assays include a cell line that naturally expresses BCMA or is engineered to express BCMA. The cell line that is engineered to express BCMA is a cell line that does not express BCMA under normal conditions but expresses BCMA after transfecting DNA encoding BCMA into the cells.

### III. Fusions and conjugates

The present invention provides a fusion or a conjugate comprising the antibody of the present invention. The antibody of the present invention may be fused or conjugated to a heterologous molecule to produce a fusion or a conjugate.

In some embodiments, the antibody polypeptide of the present invention may be fused or conjugated to one or more heterologous molecules, wherein the heterologous molecules include, but are not limited to, proteins/polypeptides/peptides, markers, drugs, and cytotoxic agents. The method of fusion or conjugation of protein, polypeptide or peptide or chemical molecule to an antibody is known in the art. See, for example, US 5,336,603, US 5,622,929, and EP 367,166.

In one embodiment, the antibody of the present invention is recombined and fused with a heterologous protein or polypeptide or peptide to form a fusion protein. In another embodiment, the antibody of the present invention is conjugated to a protein molecule or a non-protein molecule to produce a conjugate.

In some embodiments, the antibody of the present invention may be fused or conjugated to a heterologous molecule in the form of a full-length antibody or an antibody fragment.

A linker may be used to covalently link different entities in the fusion and/or the conjugate of the invention. The linker includes a chemical linker or a single chain peptide linker. In some embodiments, the antibody of the present invention is fused to other peptide segments or proteins via a peptide linker. In some embodiments, the antibody of the present invention is conjugated to other molecules, such as a marker or a drug molecule, via a chemical linker.

The peptide linker of the invention comprises a peptide consisting of amino acid residues. Such a linker peptide is usually flexible, allowing the antigen binding portion linked thereto to move independently. The length of the linker peptide can be easily determined by those skilled in the art according to the actual situation, such as at least 4-15 amino acids, or longer, such as about 20-25 amino acids.

### IV. Methods and compositions for diagnosis and detection

The present invention provides a use of the anti-BCMA antibody, the fusion or the conjugate of the invention in diagnosis and detection. Any anti-BCMA antibody, fusion or conjugate provided herein can be used to detect the presence of human BCMA in biological samples.

The term "detection" as used herein includes quantitative or qualitative detections. Exemplary detection methods include, but are not limited to, immunohistochemistry, immunocytochemistry, flow cytometry (e.g., FACS), magnetic beads complexed with antibody molecules, ELISA assay, and PCR technology (e.g., RT-PCR). In some embodiments, the biological sample includes body fluids, cells, or tissues. In certain embodiments, the biological sample is blood, serum, or other liquid samples of biological origin.

In one embodiment, provided is the anti-BCMA antibody, the fusion or the conjugate for use in a diagnostic or detection method. In a further aspect, a method for detecting the presence of BCMA in a biological sample is provided. In some embodiments, the method comprises contacting a biological sample with the anti-BCMA antibody, the fusion or the conjugate as described herein under conditions that allow binding of the anti-BCMA antibody, the fusion or the conjugate to BCMA, and detecting whether a complex is formed between the anti-BCMA antibody, the fusion or the conjugate and BCMA. The method may be an *in vitro* or *in vivo* method.

In one embodiment, the anti-BCMA antibody, the fusion or the conjugate is used to select a subject suitable for treatment with the anti-BCMA antibody, for example, when BCMA is a biomarker for patient selection. Exemplary diseases that can be diagnosed using the antibody, the fusion, or the conjugate of the invention include B cell related diseases, such as multiple myeloma. In some embodiments, a method for delaminating a patient with multiple myeloma (MM) using the antibody, the fusion or the conjugate of the invention is provided. The method comprises determining whether the patient's B cells, preferably malignant B cells, express BCMA protein on the surface of the B cells, and if BCMA protein is expressed on the surface of the B cells, then the patient will probably respond to and use a therapeutic agent (such as anti-BCMA antibody) targeting BCMA for treatment. In some embodiments, the anti-BCMA antibody may be conjugated to a diagnostic agent or a detectable agent. In some embodiments, the present invention provides a kit for diagnosis or detection that comprises any anti-BCMA antibody, fusion or conjugate of the invention.

### V. Methods and compositions for treatment

The present invention provides a method for treating B cell related diseases, comprising administering an effective amount of the antibody or antigen-binding fragment thereof, or the fusion or the conjugate of the present invention to a subject.

B cell related diseases are diseases related to abnormal B cell activity, including but not limited to B cell malignant tumors, plasma cell malignant tumors, and autoimmune diseases. Exemplary diseases that can be treated with the BCMA antibody include, for example, multiple myeloma, non-Hodgkin's lymphoma, B cell proliferation with uncertain malignant potential, lymphomatoid granulomatosis, post-transplant lymphoproliferative diseases, immune regulatory diseases, rheumatoid arthritis, myasthenia gravis, idiopathic thrombocytopenic purpura, antiphospholipid syndrome, Chagas disease, Graves' disease, Wegener's granulomatosis, polyarteritis nodosa, Schegren syndrome, pemphigus vulgaris, scleroderma, multiple sclerosis, ANCA-associated vasculitis, Goodpasture's disease, Kawasaki disease, autoimmune hemolytic anemia, and rapidly progressive glomerulonephritis, heavy chain disease, primary or immune cell related amyloidosis, or monoclonal gammopathy of undetermined significance, systemic lupus erythematosus, rheumatoid arthritis.

In some embodiments, the antibody, the fusion and the conjugate of the present invention are used to treat human B cell related diseases, such as B cell malignant tumors, preferably multiple myeloma (MM) or non-Hodgkin's lymphoma (NHL). In some embodiments, the anti-BCMA antibody, the fusion and the conjugate of the present invention have anti-tumor effects, including but not limited to, for example, reducing tumor volume, reducing the number of tumor cells, reducing tumor cell proliferation or reducing tumor cell survival.

It can be understood that the BCMA antibody, the fusion and the conjugate of the present invention can be used in combination with other therapeutic forms for the treatment of the above diseases, such as tumors. The other treatment forms include therapeutic agents, radiotherapy, chemotherapy, transplantation, immunotherapy, etc. In some embodiments, the antibody molecule, the fusion and the conjugate of the present invention are used in combination with other therapeutic agents. Exemplary therapeutic agents include cytokines, growth factors, steroids, NSAID, DMARD, anti-inflammatory agents, chemotherapy agents, radiotherapy agents, therapeutic antibodies or other active agents and adjuvants, such as anti-tumor drugs.

The following examples are described to assist in understanding the present invention.

The following examples are described to assist in understanding the present invention. The examples are not intended to be and should not be interpreted in any way as limiting the protection scope of the present invention.

### EXAMPLES

### Example 1 Production and expression of antibodies having an improved affinity for BCMA

The heavy chain variable region and light chain variable region sequences (VH sequence shown in SEQ ID NO: 9 and VL sequence shown in SEQ ID NO: 18) of anti-BCMA parent antibody ADI-34861, and the heavy chain variable region and light chain variable region sequences (VH sequence shown in SEQ ID NO: 63 and VL sequence shown in SEQ ID NO: 72) of anti-BCMA parent antibody ADI-34857, were obtained from the international application No. PCT/CN2019/074419 (BCMA antibody related patent).

In order to obtain antibodies with improved BCMA affinity, the Example designed affinity variants derived from anti-BCMA parent antibodies ADI-34861 and ADI-34857, and tested the functions of the varaints.

Using the standard protocol (Silacci et al., (2005), Proteomics 5, 2340-50), the generation of affinity mature Fabs derived from parent antibodies ADI-34861 and ADI-34857 was performed by phage display. For parent antibody ADI-34861, after several rounds of panning, antibody ADI-38491 (with VH sequence shown in SEQ ID NO: 27 and VL sequence shown in SEQ ID NO: 36) and antibody ADI-38497 (with VH sequence shown in SEQ ID NO: 45 and VL sequence shown in SEQ ID NO: 54) were prelimitarily obtained as having improved affinity. For parent antibody ADI-34857, after several rounds of panning, antibody ADI-38481 (with VH sequence shown in SEQ ID NO: 81 and VL sequence shown in SEQ ID NO: 90) and antibody ADI-38484 (with VH sequence shown in SEQ ID NO: 99 and VL sequence shown in SEQ ID NO: 108) were prelimitarily obtained as having improved affinity.

The nucleotide sequence (SEQ ID NO: 26) encoding the heavy chain variable region and the nucleotide sequence (SEQ ID NO: 35) encoding the light chain variable region of antibody ADI-38491, the nucleotide sequence (SEQ ID NO: 44) encoding the heavy chain variable region and the nucleotide sequence (SEQ ID NO: 53) encoding the light chain variable region of antibody ADI-38497, the nucleotide sequence (SEQ ID NO: 80) encoding the heavy chain variable region and the nucleotide sequence (SEQ ID NO: 89) encoding the light chain variable region of antibody ADI-38481, the nucleotide sequence (SEQ ID NO: 98) encoding the heavy chain variable region and the nucleotide sequence (SEQ ID NO: 107) encoding the light chain variable region of antibody ADI-38484, were respectively constructed in a modified eukaryotic expression vector plasmid pcDNA3.3 (Invitrogen) comprising light and heavy chain constant region fragments. The full-length heavy chain and light chain of each antibody were co-expressed in CHO-K cells by using ExpiCHO transient expression system (Thermo Fisher, A29133) according to the manufacturer's instructions, and then purified by protein A affinity chromatography to obtain antibody ADI-38491, antibody ADI-38497, antibody ADI-38481 and antibody ADI-38484.

### Example 2 Detection of antibody affinity by Fortebio

The Octet QKe system instrument of Fortebio Company was used to measure the antibody affinity, by the method of capturing the Fc segment of the antibody using an anti-human Fc (AHC) capture antibody as a biological probe. The specific operations were as follows.

The antibody ADI-34861, antibody ADI-38491, antibody ADI-38497, antibody ADI-34857, antibody ADI-38481 and antibody ADI-38484 were respectively diluted to 4 µg/ml with PBS buffer, and flew through the surface of AHC probe (Cat: 18-0015, PALL) for 120s. BCMAs (60nM) from human, cynomolgus monkey and mouse were used as mobile phase, with association time of 180s and dissociation time of 180s. After the experiment, the response value of blank control (PBS buffer) was deducted, the 1: 1 Langmuir binding mode fitting was performed using software to calculate the kinetic constants of antigen-antibody binding. The kinetic constants were shown in Table 1 below.

The results showed that compared with the corresponding parent antibody ADI-34861, the affinities of the mutant antibody ADI-38491 and the mutant antibody ADI-38497 were significantly improved; and compared with the corresponding parent antibody ADI-34857, the affinities of the mutant antibody ADI-38481 and the mutant antibody ADI-38484 were significantly improved.

**Table 1 Binding affinity of each antibody to BCMA**

| **Antibody** | **ForteBio IgG K_{D} Human BCMA-IHM (100 nM) in solution [monovalent]** | **ForteBio IgG K_{D} Human BCMA monomer (100 nM) in solution [monovalent]** | **ForteBio IgG K_{D} Cynomolgus monkey BCMA-Fc (100 nM) in solution [Avid]** | **ForteBio IgG K_{D} Mouse BCMA-Fc(100 nM) in solution [Avid]** |
|---|---|---|---|---|
| ADI-34861 | 5.14E-08 | 1.73E-08 | 2.11E-09 | 3.68E-09 |
| ADI-38491 | 2.62E-09 | 1.02E-09 | 1.27E-09 | 3.04E-09 |
| ADI-38497 | 1.02E-09 | 6.21E-10 | 6.02E-10 | 5.03E-10 |
| ADI-34857 | 4.40E-08 | 3.59E-08 | 1.47E-09 | 2.41E-09 |
| ADI-38481 | 4.96E-10 | 1.00E-10 | 8.97E-10 | 2.44E-09 |
| ADI-38484 | 5.39E-10 | 7.94E-11 | 1.07E-09 | 8.50E-10 |

### Example 3 Cell-based antibody function assay

In order to test the binding affinity of the antibody to BCMA expressed on the cell surface, three kinds of cells (NCI-H929 cells, BCMA-KO-H929 cells and human BCMA CHO-S cells) were used for determination. NCI-H929 cell (also referred to as H929 cell for short herein) (purchased from Nanjing Cobioer Biosciences Co., Ltd.) is a human multiple myeloma cell line that naturally expresses BCMA molecules on the cell surface. BCMA-KO-H929 cell is a cell line (entrusted Nanjing Genscript Biotech Corporation to construct) that is based on H929 cell and uses CRISPR-Cas9 technology to specifically target BCMA gene, causing BCMA gene to undergo frameshift mutation, and then the cell is unable to normally express BCMA molecules. Human BCMA CHO-S cells were prepared by introducing exogenous human BCMA gene into CHO-S cells. The preparation method of human BCMA CHO-S cells was as follows: a sequence encoding human BCMA (NP_001183.2, SEQ ID NO: 109) was cloned into a polyclonal site of pcDNA3.3 (Invitrogen) vector, and an expression vector expressing human BCMA was obtained; then the expression vector expressing human BCMA was introduced into CHO-S cells (ATCC) for eukaryotic expression, and CHO-S cells expressing human BCMA on the cell surface were obtained.

Human BCMA CHO-S cells or H929 cells were seeded into a 96-well plate by 1.0×10⁵ cells/well, and 10nM of each of the diluted antibodies (antibody ADI-34861, antibody ADI-38491, antibody ADI-38497, antibody ADI-34857, antibody ADI-38481 and antibody ADI-38484) was added. After incubation at 4°C for 30 minutes, cells were washed and 100 µL APC labeled goat anti-human IgG secondary antibody (Jackson ImmunoResearch Inc, Catalog # 109-136-097, Allophycocyanin (APC) AffiniPure F(ab')₂ Fragment Goat Anti-Human IgG, F(ab')₂ fragment specific) was added and incubated at 4°C for 30 minutes. Then the cells were washed and the binding of each antibody to BCMA molecules expressed on the cell surface was detected by flow cytometry (Beckman Coulter). CHO-S cells were used as negative control.

**Table 2 Binding of each antibody to BCMA expressed on the cell surface**

| **Antibody** | **CHO-S cell MFI (Median Fluorescence Intensity)** | **Human BCMA CHO-S cell MFI** | **Human BCMA CHO-S cell (FOB, Fold Over Background)** | **Human BCMA H929 cell MFI** | **Human BCMA H929 cell (FON, Fold Over Negative)** |
|---|---|---|---|---|---|
| ADI-34861 | 252 | 1001 | 4 | 1397 | 6 |
| ADI-38491 | 247 | 996 | 4 | 1098 | 5 |
| ADI-38497 | 276 | 2316 | 8 | 5671 | 26 |
| ADI-34857 | 304 | 877 | 3 | 2275 | 10 |
| ADI-38481 | 270 | 2622 | 10 | 6659 | 31 |
| ADI-38484 | 252 | 2725 | 11 | 7718 | 35 |

Table 2 showed that antibody ADI-34861, antibody ADI-38491, and antibody ADI-38497 bound to BCMA expressed on the cell surface, and the binding affinity of antibody ADI-38497 to BCMA expressed on the cell surface was significantly increased; and antibody ADI-34857, antibody ADI-38481, and antibody ADI-38484 bound to BCMA expressed on the cell surface, and the binding affinities of antibody ADI-38481 and antibody ADI-38484 to BCMA expressed on the cell surface were significantly increased.

Further, H929 cells and BCMA-KO-H929 cells were seeded into a 96-well plate by 3.0×10⁵ cells/well and diluted ADI-38497 antibody (1 µg/well) was added. No antibody was added to the negative control well. After incubation at 4°C for 30 minutes, the cells were washed and 100 µL APC labeled goat anti-human IgG secondary antibody (Jackson ImmunoResearch Inc, Catalog # 109-136-097) was added to all cell wells and incubated at 4°C for 30 minutes. The cells were then washed and the binding of antibody ADI-38497 to BCMA molecules expressed on the surface of each cell was detected by flow cytometry.

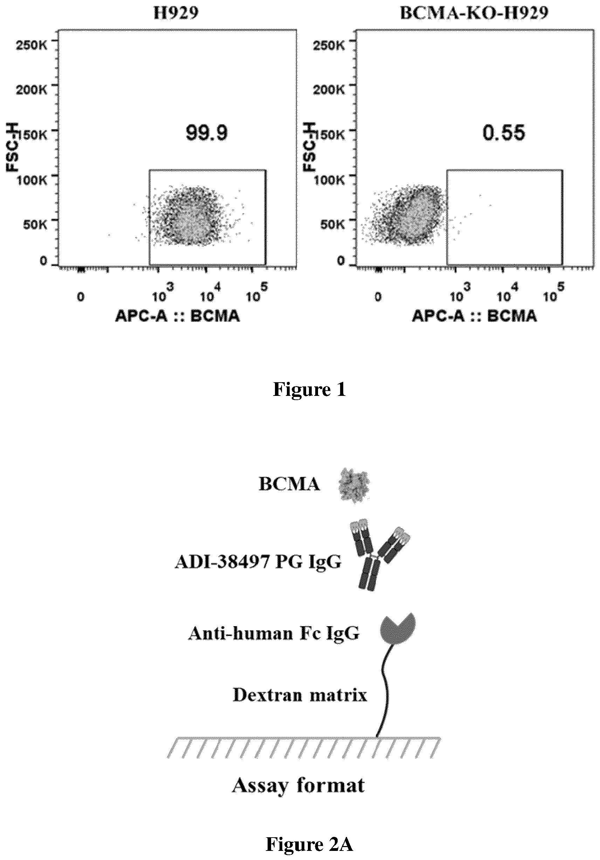

It could be seen from Figure 1 that ADI-38497 antibody only bound to H929 cells expressing BCMA antigen, but not to BCMA-KO-H929 cells with BCMA gene knockout. Therefore, ADI-38497 antibody can specifically bind to BCMA antigen.

### Example 4 Preparations of ADI-38497 WT antibody, ADI-38497 PG antibody, ADI-38484 WT antibody and ADI-38484 PG antibody, and detections of antigen-binding activity

### 4.1 Preparations of ADI-38497 WT antibody, ADI-38497 PG antibody, ADI-38484 WT antibody and ADI-38484 PG antibody

The light and heavy chain variable region sequences of BCMA antibody clone J6M0 of GSK company were obtained from US9273141B2 patent, and used in a control antibody (designated as GSK IgG).

The light and heavy chain variable region sequences of GSK IgG, ADI-38497, and ADI-38484 antibodies were synthesized using the whole gene, and inserted into pcDNA3.4 expression vectors (purchased from Shanghai Bio-Innovation Biotechnology Co., Ltd.) containing WT human IgG1 heavy chain constant region (SEQ ID NO:110) or containing P329G site-mutated human IgG1 heavy chain constant region (SEQ ID NO:111), and containing κ light chain constant region (SEQ ID NO:112). The obtained light and heavy chain expression vectors were co-transfected via PEI into HEK293 cells at a molar ratio of 2:3, and the medium supernatant was collected after 5-7 days of culture. The antibody-comprising supernatant was further purified through Protein A column and then dialyzed against PBS. The concentration was detected by reading the absorbance at 280 nm using a NanoDrop instrument, and the purity of the sample was detected by SDS-PAGE and SEC-HPLC. GSK WT antibody, GSK PG antibody; ADI-38497 WT antibody, ADI-38497 PG antibody, ADI-38484 WT antibody and ADI-38484 PG antibody were obtained.

### 4.2 Affinity assay of ADI-38497 PG antibody

The affinity of ADI-38497 PG antibody to BCMA derived from different species was detected using Biacore T200. Figure 2A showed a schematic diagram of the method for detecting antibody affinity by surface plasmon resonance (SPR).

The particular method was as follows: After coupling anti-human Fc IgG (Ab97221, Abcam) to the surface of a CM5 chip (29149603, Cytiva), the ADI-38497 PG antibody was captured on the chip surface. Affinity and kinetic constants were obtained by detecting the association and dissociation between the antibody on the chip surface and the BCMA antigen in the mobile phase. 10-fold diluted 10×HBS-EP+ (BR-1006-69, Cytiva) was used as the experimental buffer during the assay. Each cycle in the affinity detection consists of capture of the ADI-38497 PG antibody, binding of one concentration of antigen, and regeneration of the chip. The gradient diluted antigens (1.25-40 nM of antigen concentration gradients, 2-fold dilution) flew over the surface of the chip from low concentration to high concentration at a flow rate of 30 µl/min, and the association time was 180s. A suitable dissociation time (900s or 600s or 60s) was set. Finally, the chip was regenerated with 10 mM glycine-HCl, pH 1.5 (BR-1003-54, Cytiva).

The data results were analyzed by Biacore T200 analysis software (version 3.1) using a 1:1 binding model.

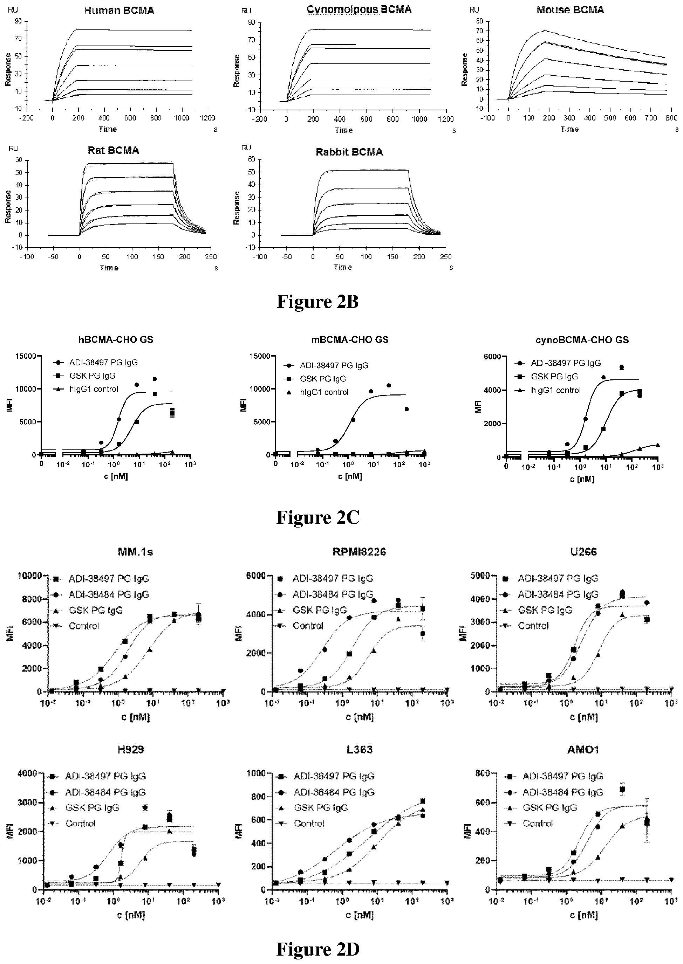

Figure 2B showed representative affinity profiles of ADI-38497 PG antibody to the recombinant human, cynomolgus monkey, mouse, rat and rabbit BCMA proteins, as measured by SPR. The results showed that ADI-38497 PG antibody could bind to BCMA proteins derived from above-mentioned different species, and the order of binding activity was human BCMA > monkey BCMA > mouse BCMA > rat BCMA > rabbit BCMA.

**Table 3 Binding affinity of ADI-38497 PG antibody to BCMA proteins from different species**

| Antigen | Antibody | ka (1/Ms) | kd (1/s) | KD (M) |
|---|---|---|---|---|
| Human BCMA | ADI-38497 PG Antibody | 2.878E+5 | 1.756E-5 | 6.101E-11 |
| cynomolgus monkey BCMA | | 3.402E+5 | 1.025E-5 | 3.012E-11 |
| Mouse BCMA | | 4.920E+5 | 8.716E-4 | 1.772E-9 |
| Rat BCMA | | 1.001E+6 | 8.314E-2 | 8.306E-8 |
| Rabbit BCMA | | 5.830E+4 | 5.309E-2 | 9.106E-7 |

### 4.3 Detection of binding activity of P329G BCMA antibody to BCMA antigens from different species

First, CHO GS cells expressing BCMA antigens from different species were prepared. Particularly, the BCMA genes from human, mouse, and cynomolgus monkey sources were synthesized and cloned into lentiviral vectors, and then lentiviruses containing BCMA genes from different species were packaged, and CHO GS cells were infected with the lentiviruses. Then CHO GS cell lines expressing BCMA antigens from different species were obtained by flow cytometry sorting, named as hBCMA-CHO GS, mBCMA-CHO GS and cynoBCMA-CHO GS cells.

Then, FACS buffer was used to dilute ADI-38497 PG antibody and GSK-derived BCMA antibody (i.e., GSK PG IgG was used as Benchmark) into antibody solutions of different concentrations with 10-fold gradient dilution, which were respectively incubated with 1E5 CHO GS cells expressing BCMA antigens from different species at 4°C for 30 minutes. After washing with FACS buffer, the cells were then incubated with Fcγ fragment-specific APC-goat anti-human IgG (Jackson ImmunoResearch, 109-136-098) at 4°C for 30 minutes. The P329G antibody bound to the cells was detected by flow cytometry, and MFI of the APC channel was analyzed. Plotting was carried out with the antibody concentration as the X axis and the APC channel MFI as the Y axis and the binding EC50 was calculated.

Figure 2C showed the binding ability of P329G BCMA antibody in different concentrations to CHO-GS cells stably expressing human BCMA, cynomolgus monkey BCMA or mouse BCMA. It could be seen from Figure 2C that ADI-38497 PG IgG antibody bound to different species derived

BCMA expressed on the cell surface, while GSK-derived BCMA antibody (Benchmark) had higher specificity to species derived BCMA, which did not recognize mouse BCMA, and this result was consistent with the SPR detection result.

**Table 4 EC50 value of P329G BCMA antibody binding to CHO-GS cells expressing BCMA derived from different species**

| Cell line | EC50(nM) of Antibody | |
|---|---|---|
| | ADI-38497 PG IgG | GSK PG IgG |
| hBCMA-CHO-GS | 1.490 | 5.002 |
| mBCMA-CHO-GS | 1.183 | 131.9 |
| cynoBCMA-CHO-GS | 1.692 | 10.01 |

### 4.4 Detection of binding activity of P329G BCMA antibody to BCMA antigen on tumor cell surface

A proper amount of tumor cells in logarithmic growth phase was taken, and washed with FACS buffer twice. ADI-38497 PG antibody, ADI-38484 PG antibody, or GSK PG IgG used as Benchmark was added. Isotype hIgG 1 antibody was added to cells used as dyeing control. Dyeing was carried out at 4°C for 30 minutes and washing was performed twice. APC-F(ab')₂ Fragment Goat Anti-Human IgG antibody was added and dyeing was performed at 4°C for 30 minutes. Cells were washed twice, then suspended with FACS buffer, and detected using flow cytometry.

Figure 2D showed the binding activities of different concentrations of P329G BCMA antibody to positive multiple myeloma cell lines MM.1s, RPMI8226, U266, H929, L363 and AMO1 expressing BCMA (MM.1s was purchased from Nanjing Cobioer Biosciences Co., Ltd., CBP60239; RPMI8226 was purchased from Nanjing Cobioer Biosciences Co., Ltd., CBP60244; U266 was purchased from Wuhan Procell Life Science&Technology Co.,Ltd., CL-0510; H929 was purchased from Nanjing Cobioer Biosciences Co., Ltd., CBP60243; L363 was purchased from Nanjing Cobioer Biosciences Co., Ltd., CBP6024; AMO1 was purchased from Nanjing Cobioer Biosciences Co., Ltd., CBP60242). ADI-38497 PG antibody, ADI-38484 PG antibody could bind to positive tumor cells expressing BCMA and exhibit antibody concentration dependency. Among the positive tumor cell lines expressing BCMA, MM.1s cells had the highest level of BCMA expression, RPMI8226, U266 and H929 cells expressed BCMA at a medium level, and L363 and AMO1 cells expressed BCMA at a low level.

**Table 5 EC50 value of P329G BCMA antibody binding to BCMA expressing positive tumor cells**

| | EC50(nM) of Antibody | | |
|---|---|---|---|
| Name of cell line | ADI-38497 PG IgG | ADI-38484 PG IgG | GSK PG IgG |
| MM.1s | 1.007 | 1.953 | 7.535 |
| RPMI8226 | 2.117 | 0.428 | 5.972 |
| U266 | 1.795 | 2.789 | 8.736 |
| H929 | ∼ 1.713 | ∼ 1.548 | ∼ 7.686 |
| L363 | 5.113 | 0.9341 | 10.39 |
| AMO1 | 2.428 | 4.250 | 12.92 |

### Example 5 Biological function detection of ADI-38497 WT antibody and ADI-38497 PG antibody

### 5.1 ADCC effector function test

PBMC cells (Peripheral Blood Mononuclear Cells) derived from donor 3 were resuscitated, resuspended in RPMI 1640 medium containing 10% fetal bovine serum, and stabilized at 37°C for 1-2 hours. The PBMCs and target cells were mixed in terms of an effector to target ratio of 25:1, and mixed with different concentrations of BCMA antibodies, cultured at 37°C for 4 hours and 24 hours respectively. The antibody mediated killing effect of PBMCs on target cells was detected using LDH detection kit (Promega, G1780). The antibody concentrations were taken as the X axis, and the cell lysis ratios were taken as the Y axis for plotting and analysis. Cells were collected at the same time, washed twice with FACS buffer, and CD3 antibody, CD56 antibody, CD16 antibody and CD107a antibody were added, of which CD107a antibody should be added in advance, and incubated with cells at 37°C for 1 hour. The above mixtures of cells and antibodies were dyed at 4 °C for 30 minutes, washed twice, suspended in FACS buffer, and detected using flow cytometry.

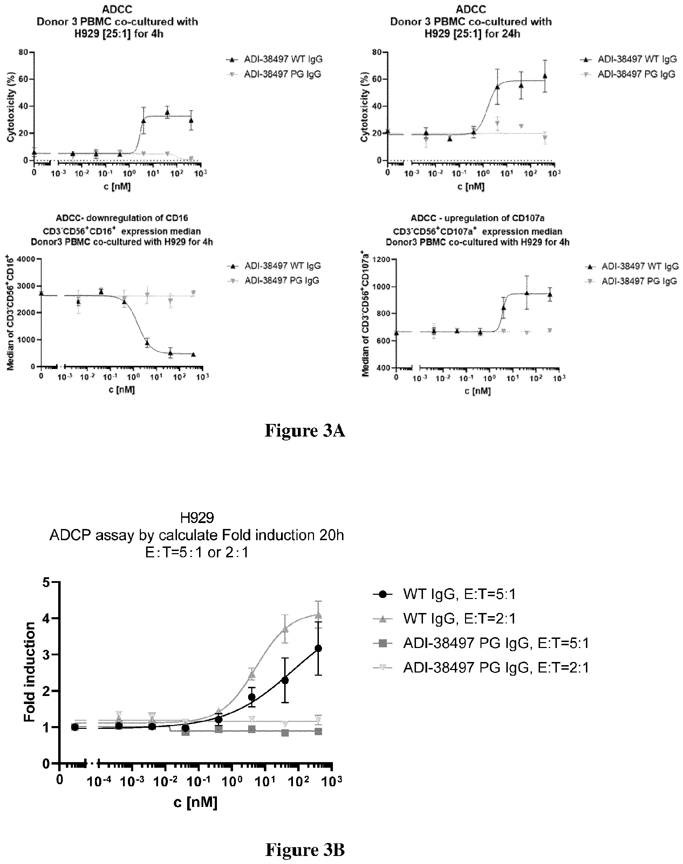

Figure 3A showed the abilities of ADI-38497 WT antibody and ADI-38497 PG antibody to mediate ADCC killing. The results showed that when testing different incubation time (4 hours, 24 hours) and using different detection indicators (cytotoxicity to target cells, influence on the expressions of CD3, CD56, CD16 and CD107a), only WT antibody could mediate the ADCC cytotoxicity killing effect on positive H929 tumor cells expressing BCMA, while P329G mutant antibody lacked the ability to mediate ADCC effect.

### 5.2 ADCP effector function test

ADCP report cell line (Promega, G9871) and H929 cells in logarithmic growth phase were taken. ADCP report cells and H929 target cells were mixed in terms of an effector to target ratio of 2:1 or 5:1, and mixed with different concentrations of BCMA antibodies, continued to culture at 37°C for 20 hours. A luciferase detection kit (Promega, E2620) was used to detect the antibody mediated and target cell dependent activation effect of report cells. The antibody concentrations were used as the X axis, and fluorescence reading changes were taken as the Y axis for plotting and analysis.

Figure 3B showed the ability of ADI-38497 WT antibody and ADI-38497 PG antibody to mediate ADCP killing. The results showed that when tested with different ratios of effector to target (2:1 or 5:1), only ADI-38497 WT antibody mediated ADCP killing effect on BCMA positive H929 tumor cells, while P329G mutant antibody lacked the ability to mediate ADCP killing effect.

### 5.3 Anti-proliferation function test of P329G mutation antibody

H929 cells and L363 cells in logarithmic growth phase were taken, and a certain number of said cells were placed in a multiple-well plate; a part of H929 cells and a part of L363 cells in logarithmic growth phase as target cells were treated with mitomycin C, and used as positive controls. Different concentrations of ADI-38497 PG antibodies were added and mixed. They were cultured at 37°C for 48 hours, 72 hours and 120 hours respectively. The proportion of living cells was detected with CellTiter-Glo (Promega, G9242). The co-incubation time was taken as the X axis and the fluorescence reading value was taken as the Y axis for plotting and analysis.

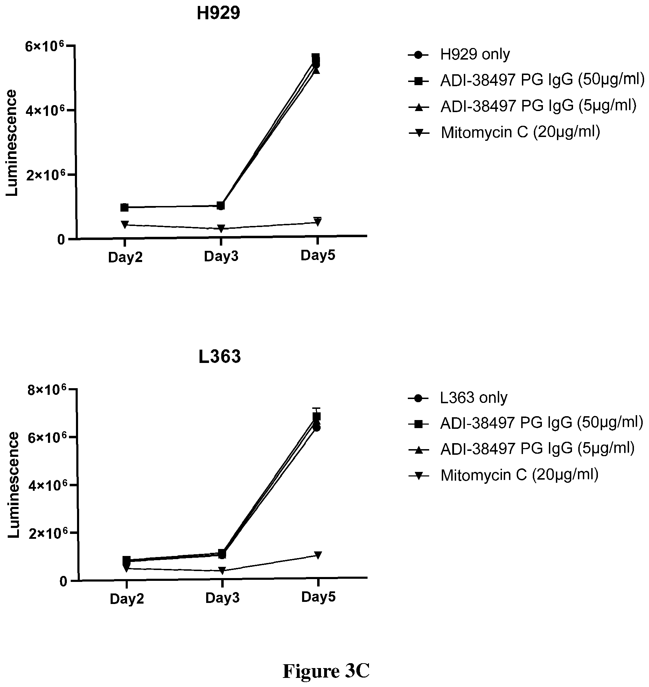

Figure 3C showed whether ADI-38497 PG antibody was capable to inhibit tumor cell proliferation. The results showed that different incubation times (48 hours, 72 hours and 120 hours) and different ADI-38497 PG antibody concentrations (5µg/ml, 50µg/ml) under test all indicated that ADI-38497 PG antibody *per se* lacked the ability to inhibit tumor cell proliferation.

### Example 6 Study on in two pharmacokinetics of ADI-38497 PG antibody

### 6.1 Antibody injection and sampling

BALB/c mice (age of 4-6 weeks, weight of 15-17g, female) were divided into three groups, i.e., 1 mg/kg of ADI-38497 PG antibody group; 10 mg/kg of ADI-38497 PG antibody group; and 200 mg/kg of ADI-38497 PG antibody group, 9 mice in each group. 1×PBS was used to dilute the antibody to 0.1 mg/mL, 1 mg/mL and 20 mg/mL respectively, and the administration volume of each mouse was 10 mL/kg, i.e., the dosage of the antibody was 1 mg/kg, 10 mg/mL, or 200 mg/mL respectively. The administration mode was intravenous injection, and the administration frequency was single. 100 µL blood samples were collected from the retroorbital venous plexus of mice 5 minutes, 30 minutes, 2 hours, 6 hours, 24 hours, 48 hours, 96 hours, 168 hours, 336 hours and 504 hours after the antibody administration, centrifugated at 3000 g, and the supernatants were taken for determination of blood drug concentration.

### 6.2 ADI-38497 PG antibody detection

A 96 well ELISA plate was coated one day in advance. BCMA antigen was diluted to 1 µg/ml using a coating solution (a bag of carbonate (Thermo, 28382) powder was dissolved in 400mL ultrapure water, then diluted to 500 mL at constant volume, and mixed well to obtain a coating solution), 100 µL per well. The plate was sealed with a plate sealing film and placed at room temperature overnight. The coating solution was removed out. The plate was patted and dried on an absorbent paper, then 300 µL washing solution was added per well, mixed under shaking for 10 seconds. After the plate was patted and dried out of washing solution, washing was repeated for 3 times. Blocking solution was added into the plate using a multichannel pipette gun, 200 µL per well. The plate was sealed with a plate sealing film and placed at room temperature for 2h. Then the plate was washed once. In standard curve (the standard curve was plotted using serially diluted BCMA antibody of known concentrations (for example, using ADI-38497 PG antibody of known concentrations to plot a standard curve) diluted, quality control samples and samples to be tested were added to the plate, 100 µL per well, incubated at room temperature for 2 h. The pre-coating solution was removed out. The plate was patted and dried on an absorbent paper, then 300 µL washing solution was added per well, mixed under shaking for 10 seconds. After the plate was patted and dried out of washing solution, washing was repeated for 3 times. Then, the washing was repeated once more. A goat anti-human IgG-Fc-HRP antibody (BETHYL) was diluted at 1: 100000, and added into each well at 100 µL, incubated at room temperature and away from light for 1h. Then the plate was washed once. TMB substrate was added to the 96-well ELISA plate at 100 µL per well. Color development was performed at room temperature and away from light for 5 minutes. 50 µL ELISA stop solution was added per well. The plate was shaked for 10 seconds, and the OD450 nm and OD620 nm values were read within 30 minutes.

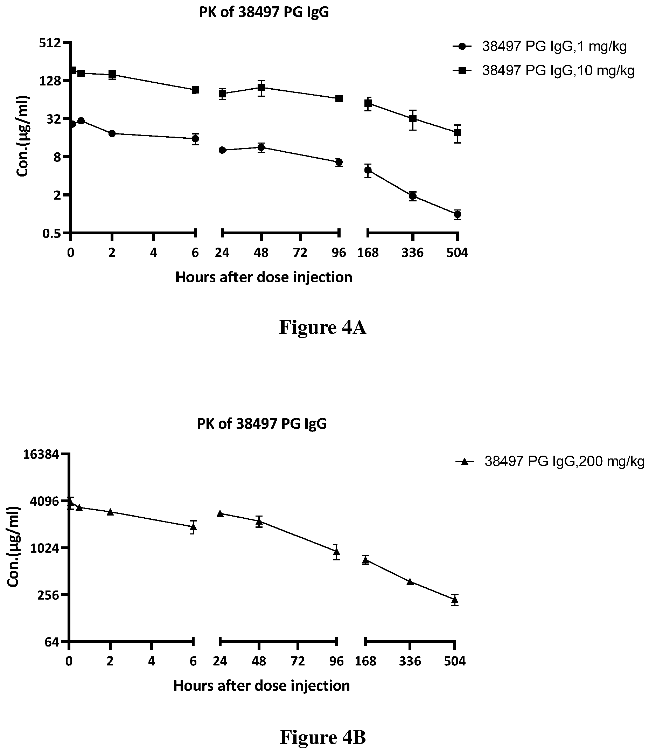

Figures 4A and 4B showed the pharmacokinetic results of ADI-38497 PG antibody in mice (hereinafter referred to as PG Ab for *in vivo* experiments in mice). After intravenous injection of ADI-38497 PG antibody at 1 mg/kg, 10 mg/kg, or 200 mg/kg, the exposure amount (Cmax and AUClast) of ADI-38497 PG antibody in serum showed a dose-dependent effect, while other pharmacokinetic parameters had no significant difference. As shown in Table 6, in 1 mg/kg of ADI-38497 PG antibody group: AUCO-inf, Cmax, CL, T1/2 were respectively 2480 µg×h/mL, 30 ug/ml, 0.40 ml/kg/h, 145 h ; in 10 mg/kg of ADI-38497 PG antibody group: AUCO-inf, Cmax, CL, T1/2 were respectively 24720 µg×h/mL, 187 ug/ml, 0.32 ml/kg/h, 219 h; in 200 mg/kg of ADI-38497 PG antibody group: AUCO-inf, Cmax, CL, T1/2 were respectively 397734 µg×h/mL, 3895 ug/ml, 0.43 ml/kg/h, 197 h. The half-life using 1 mg/kg of ADI-38497 PG antibody was slightly shorter than those using 10 mg/kg and 200 mg/kg of ADI-38497 PG antibody.

**Table 6. Pharmacokinetic test results of ADI-38497 PG antibody**

| Groups | Cₘₐₓ (µg/mL) | AUC₀₋ₜ (µg*h/mL) | AUC_{0-inf_obs} (µg*h/mL) | MRT_{0-inf_obs} (h) | t_{1/2} (h) | CL (ml/kg/h) | V_{SS} (ml/kg) |
|---|---|---|---|---|---|---|---|
| ADI-38497 PG antibody, 1 mg/kg | 30 | 2273 | 2480 | 186 | 145 | 0.40 | 75 |
| ADI-38497 PG antibody, 10 mg/kg | 187 | 24720 | 30837 | 305 | 219 | 0.32 | 99 |
| ADI-38497 PG antibody, 200 mg/kg | 3895 | 397734 | 460828 | 229 | 197 | 0.43 | 100 |

### Example 7 In vivo anti-tumor effect of ADI-38497 PG antibody

The mice were inoculated with tumor cells and treated. Particularly, H929 cells were suspended by using 1×PBS and prepared into a cell suspension with a cell density of 5 × 10⁶ cells/mL. NOG mice (age of 4-6 weeks, weight of 15-17g, female) were shaved on the right back, then subcutaneously injected with H929 cell suspension in a volume of 0.2 mL/mouse, i.e., the inoculation amount was 1 × 10⁶ cells/mouse. Seven days after tumor cell inoculation, mice with tumor volume of 50.82-104.36 mm³ were divided into PBS vehicle group and ADI-38497 PG antibody group (herein also referred to as "PG Ab group"), with seven mice in each group. After grouping, the antibody was administered on the 7th day, with the administration volume of 10 mL/kg for each mouse, the administration frequency was once a week, and the administration mode was intraperitoneal injection. The body weight, maximum long axis (L) and maximum wide axis (W) of tumor tissue were monitored twice a week.

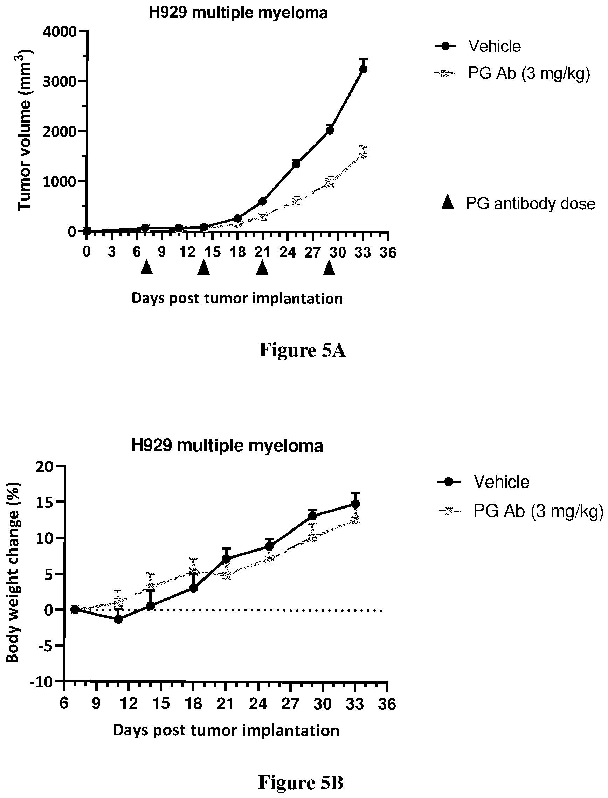

Figure 5A showed the therapeutic effect of ADI-38497 PG antibody on tumor-bearing immunodeficient mice, which had been subcutaneously inoculated with human BCMA expressing positive H929 tumor cells. The results showed that in the H929 tumor model with high expression of BCMA, the administration of PG antibody brought about significant anti-tumor effect.

Figure 5B showed the body weight changes of mice in the experiment. The results showed that in the H929 tumor model with high expression of BCMA, the body weight of mice had no significant change after the administration of the PG antibody.

Therefore, ADI-38497 PG antibody had a significant effect against BCMA high-expression tumor, and had no significant toxic and side effects.

### Example 8 In vivo anti-systemic tumor effect of ADI-38497 PG antibody

First, H929-luc cells were prepared. Particularly, H929 cells (purchased from Nanjing Cobioer Biosciences Co., Ltd.) were used to package lentiviruses comprising GFP-luciferase gene, then the obtained lentiviruses were used to infect H929 cells. Subsequently, H929-luc cell line with dual expression of GFP and luciferase was obtained through flow cytometry sorting.

Next, H929-luc cells were resuspended by using 1 × PBS and prepared into a cell suspension with a cell density of 25 × 10⁶ cells/mL. NOG mice (age of 4-6 weeks, weight of 15-17g, female) were injected with H929-luc cell suspension via tail vein, in an injection volume of 0.2 mL per mouse. Fourteen days after tumor cells were inoculated, the substrate D-Luciferin (15 mg/mL) was intraperitoneally injected in a volume of 10 mL/kg/mouse. 10 minutes after the substrate injection, IVIS Spectrum was used for imaging analysis. Mice with the fluorescent signal at 1.17 × 10⁷- 1.43 × 10⁸ photons/sec were divided into vehicle group, 0.3 mg/kg PG Ab group, 3 mg/kg PG Ab group, 6-7 mice in each group. Antibody concentrations of 0.03 mg/mL and 0.3 mg/mL were prepared respectively. After grouping, antibody administration was started on the 14th day. The administration volume of each mouse was 10 mL/kg, the administration frequency was once a week, and the administration mode was intraperitoneal injection.

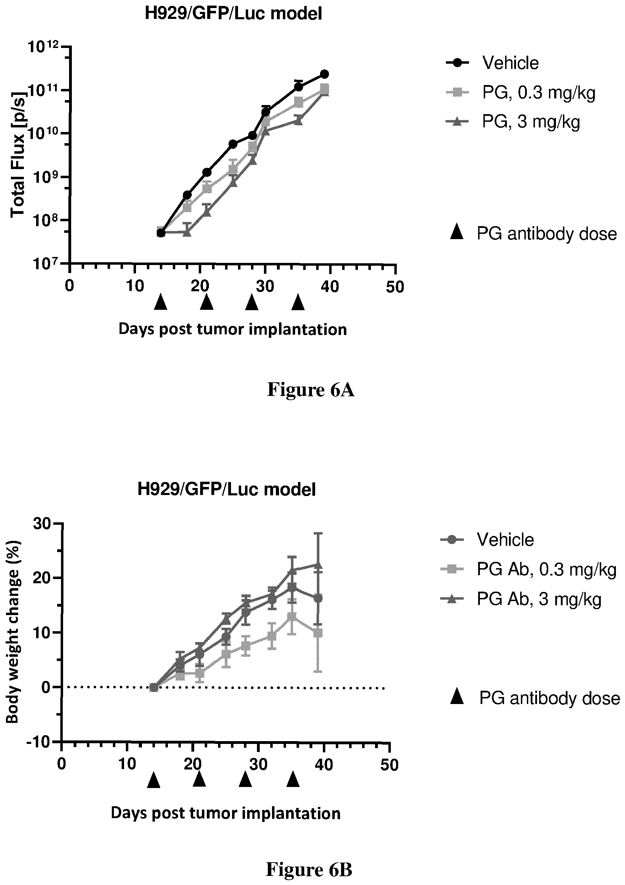

Figure 6A showed the anti-tumor effects of different doses of PG antibody on the immunodeficient tumor-bearing mice, which had been inoculated with human H929-Luc tumor cells via the tail vein.

The results showed that in the systemic tumor model, anti-tumor effect appeared in about one week after the administration of the PG antibody, and in dose-dependent manner; and the pharmaceutical effect maintained for 2 weeks, then gradually decreased.

Figure 6B showed the body weight changes of mice in the above experiment. The results showed that during the treatment period, the body weight of each treatment group increased steadily, indicating that the PG antibody treatment did not induce an obvious toxicity.

### Example 9 In vivo toxicology study of ADI-38497 PG antibody

The mice were inoculated with tumor cells and treated. Particularly, H929 cells were reususpended by using 1 × PBS and prepared into a cell suspension with a cell density of 5 × 10⁶ cells/mL. NOG mice (age of 4-6 weeks, weight of 15-17g, female) were shaved on the right back, then subcutaneously injected with H929 cell suspension of 5 × 10⁶ cells/mL in an injection volume of 0.2 mL/mouse. Six days after tumor cell inoculation, mice with tumor volume of 38.49-104.77 mm³ were grouped. As shown in Table 7, the mice were grouped into tumor-free vehicle group, tumor-bearing vehicle group and PG Ab group, with 24 mice in each group. The antibody concentration of 1 mg/mL was prepared. After grouping, the antibody was administered, in an administration volume of 10 mL/kg mouse. The administration frequency was once a week, the administrations were performed three times, in an administration mode of intraperitoneal injection. The body weight, maximum long axis (L) and maximum wide axis (W) of tumor tissue were monitored twice a week. Peripheral bloods from 4 mice in each group were taken before the first time of antibody administration, before the third time of antibody administration and at the end point of the experiment, for hematological assay and blood biochemical assay.

**Table 7 Treatment and Dosage of Each Group**

| Groups | Cell line | Treatment | Concentration |
|---|---|---|---|
| 1 | No tumor bearing | Vehicle | N/A |
| 2 | H929 | Vehicle | N/A |
| 3 | H929 | PG antibody | 10 mg/kg (QW×3) |

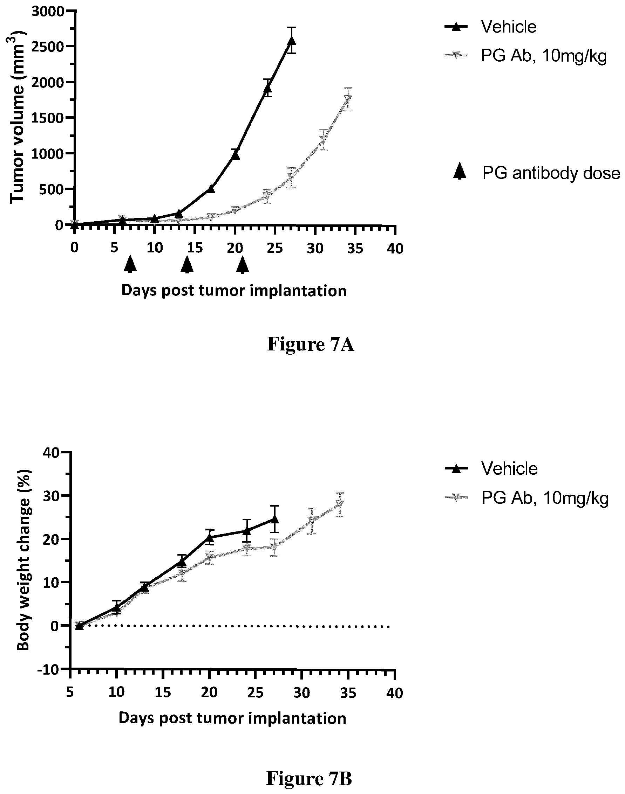

Figure 7A showed the therapeutic effect of PG antibody on the immunodeficient tumor-bearing mice, which had been inoculated subcutaneously with human H929 tumor cells. The results showed that PG antibody had an anti-tumor effect.

Figure 7B showed the body weight changes of mice in this experiment. The results showed that during the treatment with PG antibody, there was no significant change in body weight compared with the control mice, indicating that PG antibody did not induce a significant toxic reaction.

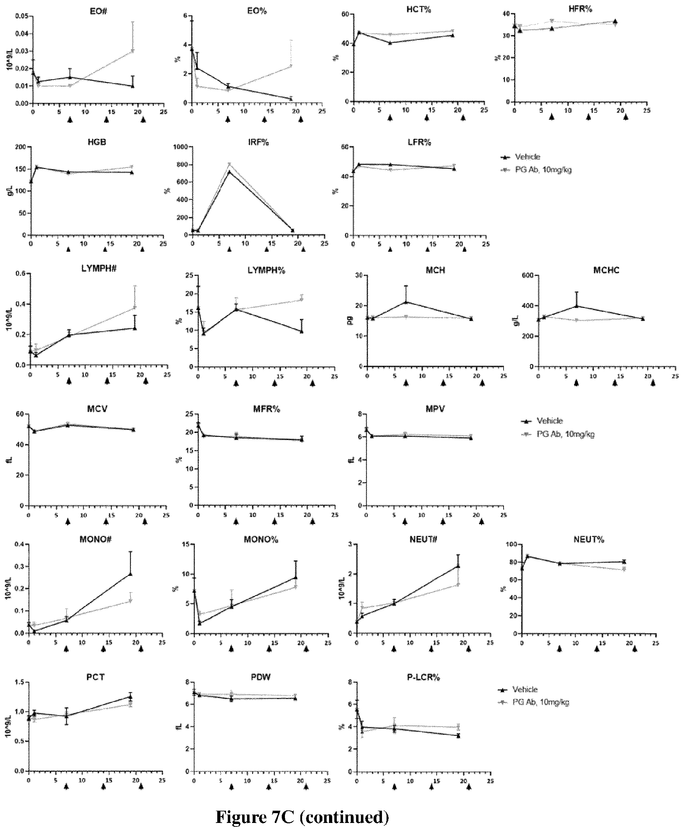

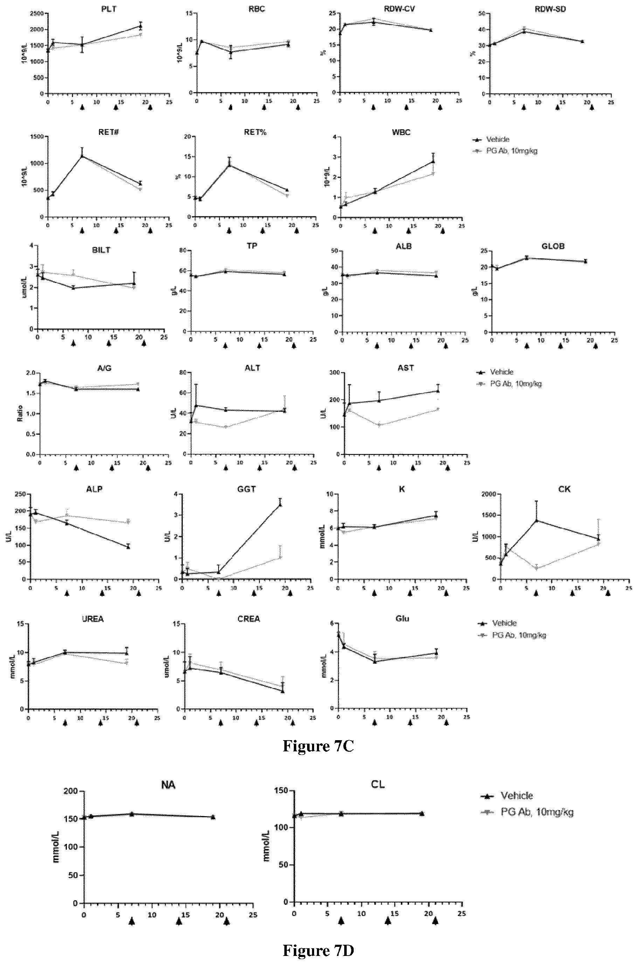

Figure 7C and Figure 7D showed the results from hematological tests and blood biochemical tests of mice in the above experiment. The results showed that during the treatment with PG antibody, there was no significant change in hematological and blood biochemical indexes of mice compared with those of the control mice, indicating that no toxic reaction was caused by the treatment with the PG antibody.

Exemplary embodiments of the present invention are described above. Those skilled in the art should recognize that these disclosures are only exemplary, and various other substitutions, adaptations, and modifications may be made to the disclosure without departing from the scope of the present invention. Therefore, the present invention is not limited to the specific embodiments exemplified herein.

### Sequence Listing

| SEQ ID NO: | Description | Amino acid sequence / nucleotide sequence |
|---|---|---|
| 1 | ADI-34861 parent antibody VH CDR1 | GSISSSSYYWG |
| 2 | ADI-34861 parent antibody VH CDR2 | SISYSGSTYYNPSLKS |
| 3 | ADI-34861 parent antibody VH CDR3 | ARDRGDTILDV |
| 4 | ADI-34861 parent antibody VHFR1 | QLQLQESGPGLVKPSETLSLTCTVSG |
| 5 | ADI-34861 parent antibody VH FR2 | WIRQPPGKGLEWIG |
| 6 | ADI-34861 parent antibody VH FR3 | RVTISVDTSKNQFSLKLSSVTAADTAVYYC |
| 7 | ADI-34861 parent antibody VH FR4 | WGQGTMVTVSS |
| 8 | ADI-34861 parent antibody VH DNA | CAGCTGCAGCTGCAGGAGTCGGGCCCAGGACTGGTGA AGCCTTCGGAGACCCTGTCCCTCACCTGCACTGTCTCT GGTGGCTCCATCAGCAGTAGTAGTTACTACTGGGGCT GGATCCGCCAGCCCCCAGGGAAGGGGCTGGAGTGGA TTGGGAGTATCTCCTATAGTGGGAGCACCTACTACAA CCCGTCCCTCAAGAGTCGAGTCACCATATCCGTAGAC ACGTCCAAGAACCAGTTCTCCCTGAAGCTGAGTTCTG TGACCGCCGCAGACACGGCGGTGTACTACTGCGCCAG AGATCGTGGAGACACCATACTAGACGTATGGGGTCAG GGTACAATGGTCACCGTCTCCTCA |
| 9 | ADI-34861 parent antibody VH amino acid | QLQLQESGPGLVKPSETLSLTCTVSGGSISSSSYYWGWIR QPPGKGLEWIGSISYSGSTYYNPSLKSRVTISVDTSKNQF SLKLSSVTAADTAVYYCARDRGDTILDVWGQGTMVTV SS |
| 10 | ADI-34861 parent antibody VL CDR1 | RASQSISRYLN |
| 11 | ADI-34861 parent antibody VL CDR2 | AASSLQS |
| 12 | ADI-34861 parent antibody VL CDR3 | QQKYFDIT |
| 13 | ADI-34861 parent antibody VL FR1 | DIQMTQSPSSLSASVGDRVTITC |
| 14 | ADI-34861 parent antibody VL FR2 | WYQQKPGKAPKLLIY |
| 15 | ADI-34861 parent antibody VL FR3 | GVPSRFSGSGSGTDFTLTISSLQPEDFATYYC |
| 16 | ADI-34861 parent antibody VL FR4 | FGGGTKVEIK |
| 17 | ADI-34861 parent antibody VL DNA | GACATCCAGATGACCCAGTCTCCATCCTCCCTGTCTGC ATCTGTAGGAGACAGAGTCACCATCACTTGCCGGGCA AGTCAGAGCATTAGCAGATATTTAAATTGGTATCAGC AGAAACCAGGGAAAGCCCCTAAGCTCCTGATCTATGC TGCATCCAGTTTGCAAAGTGGGGTCCCATCAAGGTTC AGTGGCAGTGGATCTGGGACAGATTTCACTCTCACCA TCAGCAGTCTGCAACCTGAAGATTTTGCAACTTACTAC TGTCAGCAAAAATACTTCGACATCACTTTTGGCGGAG GGACCAAGGTTGAGATCAAA |
| 18 | ADI-34861 parent antibody VL amino acid | DIQMTQSPSSLSASVGDRVTITCRASQSISRYLNWYQQKP GKAPKLLIYAASSLQSGVPSRFSGSGSGTDFTLTISSLQPE DFATYYCQQKYFDITFGGGTKVEIK |
| 19 | ADI-38491 antibody VH CDR1 | GSIVSSSYYWT |
| 20 | ADI-38491 antibody VH CDR2 | SISIAGSTYYNPSLKS |
| 21 | ADI-38491 antibody VH CDR3 | ARDRGDTILDV |
| 22 | ADI-38491 VHFR1 | QLQLQESGPGLVKPSETLSLTCTVSG |
| 23 | ADI-38491 antibody VH FR2 | WIRQPPGKGLEWIG |
| 24 | ADI-38491 antibody VH FR3 | RVTISVDTSKNQFSLKLSSVTAADTAVYYC |
| 25 | ADI-38491 antibody VH FR4 | WGQGTMVTVSS |
| 26 | ADI-38491 antibody VH DNA | CAGCTGCAGCTGCAGGAGTCGGGCCCAGGACTGGTGA AGCCTTCGGAGACCCTGTCCCTCACCTGCACTGTCTCT GGTGGCTCCATCGTCAGTAGTAGTTACTACTGGACCT GGATCCGCCAGCCCCCAGGGAAGGGGCTGGAGTGGA TTGGGAGTATCTCCATAGCAGGGAGCACCTACTACAA CCCGTCCCTCAAGAGTCGAGTCACCATATCCGTAGAC ACGTCCAAGAACCAGTTCTCCCTGAAGCTGAGTTCTG TGACCGCCGCAGACACGGCGGTGTACTACTGCGCCAG AGATCGTGGAGACACCATACTAGACGTATGGGGTCAG GGTACAATGGTCACCGTCTCCTCA |
| 27 | ADI-38491 antibody VH amino acid | QLQLQESGPGLVKPSETLSLTCTVSGGSIVSSSYYWTWIR QPPGKGLEWIGSISIAGSTYYNPSLKSRVTISVDTSKNQFS LKLSSVTAADTAVYYCARDRGDTILDVWGQGTMVTVS S |
| 28 | ADI-38491 antibody VL CDR1 | RASQSISRYLN |
| 29 | ADI-38491 antibody VL CDR2 | AASSLQS |
| 30 | ADI-38491 antibody VL CDR3 | QQKYFDIT |
| 31 | ADI-38491 antibody VL FR1 | DIQMTQSPSSLSASVGDRVTITC |
| 32 | ADI-38491 antibody VL FR2 | WYQQKPGKAPKLLIY |
| 33 | ADI-38491 antibody VL FR3 | GVPSRFSGSGSGTDFTLTISSLQPEDFATYYC |
| 34 | ADI-38491 antibody VL FR4 | FGGGTKVEIK |
| 35 | ADI-38491 antibody VL DNA | GACATCCAGATGACCCAGTCTCCATCCTCCCTGTCTGC ATCTGTAGGAGACAGAGTCACCATCACTTGCCGGGCA AGTCAGAGCATTAGCAGATATTTAAATTGGTATCAGC AGAAACCAGGGAAAGCCCCTAAGCTCCTGATCTATGC TGCATCCAGTTTGCAAAGTGGGGTCCCATCAAGGTTC AGTGGCAGTGGATCTGGGACAGATTTCACTCTCACCA TCAGCAGTCTGCAACCTGAAGATTTTGCAACTTACTAC TGTCAGCAAAAATACTTCGACATCACTTTTGGCGGAG GGACCAAGGTTGAGATCAAA |
| 36 | ADI-38491 antibody VL amino acid | DIQMTQSPSSLSASVGDRVTITCRASQSISRYLNWYQQKP GKAPKLLIYAASSLQSGVPSRFSGSGSGTDFTLTISSLQPE DFATYYCQQKYFDITFGGGTKVEIK |
| 37 | ADI-38497 antibody VH CDR1 | GSIVSSSYYWT |
| 38 | ADI-38497 antibody VH CDR2 | SISIAGSTYYNPSLKS |
| 39 | ADI-38497 antibody VH CDR3 | ARDRGDQILDV |
| 40 | ADI-38497 antibody VHFR1 | QLQLQESGPGLVKPSETLSLTCTVSG |
| 41 | ADI-38497 antibody VH FR2 | WIRQPPGKGLEWIG |
| 42 | ADI-38497 antibody VH FR3 | RVTISVDTSKNQFSLKLSSVTAADTAVYYC |
| 43 | ADI-38497 antibody VH FR4 | WSQGTMVTVSS |
| 44 | ADI-38497 antibody VH DNA | CAGCTGCAGCTGCAGGAGTCGGGCCCAGGACTGGTGA AGCCTTCGGAGACCCTGTCCCTCACCTGCACTGTCTCT GGTGGCTCCATCGTCAGTAGTAGTTACTACTGGACCT GGATCCGCCAGCCCCCAGGGAAGGGGCTGGAGTGGA TTGGGAGTATCTCCATAGCAGGGAGCACCTACTACAA CCCGTCCCTCAAGAGTCGAGTCACCATATCCGTAGAC ACGTCCAAGAACCAGTTCTCCCTGAAGCTGAGTTCTG TGACCGCCGCAGACACGGCGGTGTACTACTGCGCCAG AGATCGTGGAGACCAGATTCTAGACGTATGGAGTCAG GGTACAATGGTCACCGTCTCCTCA |
| 45 | ADI-38497 antibody VH amino acid | QLQLQESGPGLVKPSETLSLTCTVSGGSIVSSSYYWTWIR QPPGKGLEWIGSISIAGSTYYNPSLKSRVTISVDTSKNQFS LKLSSVTAADTAVYYCARDRGDQILDVWSQGTMVTVSS |
| 46 | ADI-38497 antibody VL CDR1 | RASQSISRYLN |
| 47 | ADI-38497 antibody VL CDR2 | AASSLQS |
| 48 | ADI-38497 antibody VL CDR3 | QQKYFDIT |
| 49 | ADI-38497 antibody VL FR1 | DIQMTQSPSSLSASVGDRVTITC |
| 50 | ADI-38497 antibody VL FR2 | WYQQKPGKAPKLLIY |
| 51 | ADI-38497 | GVPSRFSGSGSGTDFTLTISSLQPEDFATYYC |
| | antibody VL FR3 | |
| 52 | ADI-38497 antibody VL FR4 | FGGGTKVEIK |
| 53 | ADI-38497 antibody VL DNA | GACATCCAGATGACCCAGTCTCCATCCTCCCTGTCTGC ATCTGTAGGAGACAGAGTCACCATCACTTGCCGGGCA AGTCAGAGCATTAGCAGATATTTAAATTGGTATCAGC AGAAACCAGGGAAAGCCCCTAAGCTCCTGATCTATGC TGCATCCAGTTTGCAAAGTGGGGTCCCATCAAGGTTC AGTGGCAGTGGATCTGGGACAGATTTCACTCTCACCA TCAGCAGTCTGCAACCTGAAGATTTTGCAACTTACTAC TGTCAGCAAAAATACTTCGACATCACTTTTGGCGGAG GGACCAAGGTTGAGATCAAA |
| 54 | ADI-38497 antibody VL amino acid | DIQMTQSPSSLSASVGDRVTITCRASQSISRYLNWYQQKP GKAPKLLIYAASSLQSGVPSRFSGSGSGTDFTLTISSLQPE DFATYYCQQKYFDITFGGGTKVEIK |
| 55 | ADI-34857 parent antibody VH CDR1 | GTFSNYAIS |
| 56 | ADI-34857 parent antibody VH CDR2 | GIIPIFGTANYAQKFQG |
| 57 | ADI-34857 parent antibody VH CDR3 | ARGRGYYS SWLFDI |
| 58 | ADI-34857 parent antibody VHFR1 | QVQLVQSGAEVKKPGSSVKVSCKASG |
| 59 | ADI-34857 parent antibody VH FR2 | WVRQAPGQGLEWMG |
| 60 | ADI-34857 parent antibody VH FR3 | RVTITADESTSTAYMELSSLRSEDTAVYYC |
| 61 | ADI-34857 parent antibody VH FR4 | WGQGTMVTVSS |
| 62 | ADI-34857 parent antibody VH DNA | CAGGTGCAGCTGGTGCAGTCTGGGGCTGAGGTGAAGA AGCCTGGGTCCTCGGTGAAGGTCTCCTGCAAGGCTTC TGGAGGCACCTTCAGCAACTATGCTATCAGCTGGGTG CGACAGGCCCCTGGACAAGGGCTTGAGTGGATGGGA GGGATCATCCCTATCTTTGGTACAGCAAACTACGCAC AGAAGTTCCAGGGCAGAGTCACGATTACCGCGGACGA ATCCACGAGCACAGCCTACATGGAGCTGAGCAGCCTG AGATCTGAGGACACGGCGGTGTACTACTGCGCCAGAG GCAGGGGATACTACTCAAGCTGGCTATTCGACATATG GGGTCAGGGTACAATGGTCACCGTCTCCTCA |
| 63 | ADI-34857 parent antibody | QVQLVQSGAEVKKPGSSVKVSCKASGGTFSNYAISWVR QAPGQGLEWMGGIIPIFGTANYAQKFQGRVTITADESTS |
| | VH amino acid | TAYMELSSLRSEDTAVYYCARGRGYYSSWLFDIWGQGT MVTVSS |
| 64 | ADI-34857 parent antibody VL CDR1 | QASQDITNYLN |
| 65 | ADI-34857 parent antibody VL CDR2 | DASNLET |
| 66 | ADI-34857 parent antibody VL CDR3 | QQAFDLIT |
| 67 | ADI-34857 parent antibody VL FR1 | DIQMTQSPSSLSASVGDRVTITC |
| 68 | ADI-34857 parent antibody VL FR2 | WYQQKPGKAPKLLIY |
| 69 | ADI-34857 parent antibody VL FR3 | GVPSRFSGSGSGTDFTFTISSLQPEDIATYYC |
| 70 | ADI-34857 parent antibody VL FR4 | FGGGTKVEIK |
| 71 | ADI-34857 parent antibody VL DNA | GACATCCAGATGACCCAGTCTCCATCCTCCCTGTCTGC ATCTGTAGGAGACAGAGTCACCATCACTTGCCAGGCG AGTCAGGACATTACCAACTATTTAAATTGGTATCAGC AGAAACCAGGGAAAGCCCCTAAGCTCCTGATCTACGA TGCATCCAATTTGGAAACAGGGGTCCCATCAAGGTTC AGTGGAAGTGGATCTGGGACAGATTTTACTTTCACCA TCAGCAGCCTGCAGCCTGAAGATATTGCAACATATTA CTGTCAGCAGGCCTTCGACCTCATCACTTTTGGCGGAG GGACCAAGGTTGAGATCAAA |
| 72 | ADI-34857 parent antibody VL amino acid | DIQMTQSPSSLSASVGDRVTITCQASQDITNYLNWYQQK PGKAPKLLIYDASNLETGVPSRFSGSGSGTDFTFTISSLQP EDIATYYCQQAFDLITFGGGTKVEIK |
| 73 | ADI-38481 antibody VH CDR1 | GTFSNDVIS |
| 74 | ADI-38481 antibody VH CDR2 | VIIPIFGIANYAQKFQG |
| 75 | ADI-38481 antibody VH CDR3 | ARGRGYYS SWLLDI |
| 76 | ADI-38481 antibody VHFR1 | QVQLVQSGAEVKKPGSSVKVSCKASG |
| 77 | ADI-38481 antibody VH FR2 | WVRQAPGQGLEWMG |
| 78 | ADI-38481 antibody VH FR3 | RVTITADESTSTAYMELSSLRSEDTAVYYC |
| 79 | ADI-38481 antibody VH FR4 | WGQGTMVTVSS |
| 80 | ADI-38481 antibody VH DNA | CAGGTGCAGCTGGTGCAGTCTGGGGCTGAGGTGAAGA AGCCTGGGTCCTCGGTGAAGGTCTCCTGCAAGGCTTC TGGAGGCACCTTCAGCAACGACGTCATCAGCTGGGTG CGACAGGCCCCTGGACAAGGGCTTGAGTGGATGGGA GTCATCATCCCTATCTTCGGTATAGCAAACTACGCACA GAAGTTCCAGGGCAGAGTCACGATTACCGCGGACGAA TCCACGAGCACAGCCTACATGGAGCTGAGCAGCCTGA GATCTGAGGACACGGCGGTGTACTACTGCGCCAGAGG CAGGGGATACTACTCAAGCTGGCTTTTGGACATATGG GGTCAGGGTACAATGGTCACCGTCTCCTCA |
| 81 | ADI-38481 antibody VH amino acid | QVQLVQSGAEVKKPGSSVKVSCKASGGTFSNDVISWVR QAPGQGLEWMGVIIPIFGIANYAQKFQGRVTITADESTST AYMELSSLRSEDTAVYYCARGRGYYSSWLLDIWGQGT MVTVSS |
| 82 | ADI-38481 antibody VL CDR1 | QASQDITNYLN |
| 83 | ADI-38481 antibody VL CDR2 | DASNLET |
| 84 | ADI-38481 antibody VL CDR3 | QQAFDLIT |
| 85 | ADI-38481 antibody VL FR1 | DIQMTQSPSSLSASVGDRVTITC |
| 86 | ADI-38481 antibody VL FR2 | WYQQKPGKAPKLLIY |
| 87 | ADI-38481 antibody VL FR3 | GVPSRFSGSGSGTDFTFTISSLQPEDIATYYC |
| 88 | ADI-38481 antibody VL FR4 | FGGGTKVEIK |
| 89 | ADI-38481 antibody VL DNA | GACATCCAGATGACCCAGTCTCCATCCTCCCTGTCTGC ATCTGTAGGAGACAGAGTCACCATCACTTGCCAGGCG AGTCAGGACATTACCAACTATTTAAATTGGTATCAGC AGAAACCAGGGAAAGCCCCTAAGCTCCTGATCTACGA TGCATCCAATTTGGAAACAGGGGTCCCATCAAGGTTC AGTGGAAGTGGATCTGGGACAGATTTTACTTTCACCA TCAGCAGCCTGCAGCCTGAAGATATTGCAACATATTA CTGTCAGCAGGCCTTCGACCTCATCACTTTTGGCGGAG GGACCAAGGTTGAGATCAAA |
| 90 | ADI-38481 antibody VL amino acid | DIQMTQSPSSLSASVGDRVTITCQASQDITNYLNWYQQK PGKAPKLLIYDASNLETGVPSRFSGSGSGTDFTFTISSLQP EDIATYYCQQAFDLITFGGGTKVEIK |
| 91 | ADI-38484 antibody VH CDR1 | GTFSNDVIS |
| 92 | ADI-38484 antibody VH CDR2 | VIIPIFGIANYAQKFQG |
| 93 | ADI-38484 antibody VH CDR3 | ARGRGYYS SWLHDI |
| 94 | ADI-38484 antibody VHFR1 | QVQLVQSGAEVKKPGSSVKVSCKASG |
| 95 | ADI-38484 antibody VH FR2 | WVRQAPGQGLEWMG |
| 96 | ADI-38484 antibody VH FR3 | RVTITADESTSTAYMELSSLRSEDTAVYYC |
| 97 | ADI-38484 antibody VH FR4 | WGQGTMVTVSS |
| 98 | ADI-38484 antibody VH DNA | CAGGTGCAGCTGGTGCAGTCTGGGGCTGAGGTGAAGA AGCCTGGGTCCTCGGTGAAGGTCTCCTGCAAGGCTTC TGGAGGCACCTTCAGCAACGACGTCATCAGCTGGGTG CGACAGGCCCCTGGACAAGGGCTTGAGTGGATGGGA GTCATCATCCCTATCTTCGGTATAGCAAACTACGCACA GAAGTTCCAGGGCAGAGTCACGATTACCGCGGACGAA TCCACGAGCACAGCCTACATGGAGCTGAGCAGCCTGA GATCTGAGGACACGGCGGTGTACTACTGCGCCAGAGG CAGGGGATACTACTCAAGCTGGCTGCATGACATATGG GGTCAGGGTACAATGGTCACCGTCTCCTCA |
| 99 | ADI-38484 antibody VH amino acid | QVQLVQSGAEVKKPGSSVKVSCKASGGTFSNDVISWVR QAPGQGLEWMGVIIPIFGIANYAQKFQGRVTITADESTST AYMELSSLRSEDTAVYYCARGRGYYSSWLHDIWGQGT MVTVSS |
| 100 | ADI-38484 antibody VL CDR1 | QASQDITNYLN |
| 101 | ADI-38484 antibody VL CDR2 | DASNLET |
| 102 | ADI-38484 antibody VL CDR3 | QQAFDLIT |
| 103 | ADI-38484 antibody VL FR1 | DIQMTQSPSSLSASVGDRVTITC |
| 104 | ADI-38484 antibody VL FR2 | WYQQKPGKAPKLLIY |
| 105 | ADI-38484 antibody VL FR3 | GVPSRFSGSGSGTDFTFTISSLQPEDIATYYC |
| 106 | ADI-38484 antibody VL FR4 | FGGGTKVEIK |
| 107 | ADI-38484 antibody VL DNA | GACATCCAGATGACCCAGTCTCCATCCTCCCTGTCTGC ATCTGTAGGAGACAGAGTCACCATCACTTGCCAGGCG AGTCAGGACATTACCAACTATTTAAATTGGTATCAGC AGAAACCAGGGAAAGCCCCTAAGCTCCTGATCTACGA TGCATCCAATTTGGAAACAGGGGTCCCATCAAGGTTC AGTGGAAGTGGATCTGGGACAGATTTTACTTTCACCA TCAGCAGCCTGCAGCCTGAAGATATTGCAACATATTA CTGTCAGCAGGCCTTCGACCTCATCACTTTCGGCGGA GGGACCAAGGTGGAGATCAAA |
| 108 | ADI-38484 antibody VL amino acid | DIQMTQSPSSLSASVGDRVTITCQASQDITNYLNWYQQK PGKAPKLLIYDASNLETGVPSRFSGSGSGTDFTFTISSLQP EDIATYYCQQAFDLITFGGGTKVEIK |
| 109 | Human BCMA sequence | MLQMAGQCSQNEYFDSLLHACIPCQLRCSSNTPPLTCQR YCNASVTNSVKGTNAILWTCLGLSLIISLAVFVLMFLLR KINSEPLKDEFKNTGSGLLGMANIDLEKSRTGDEIILPRG LEYTVEECTCEDCIKSKPKVDSDHCFPLPAMEEGATILVT TKTNDYCKSLPAALSATEIEKSISAR |
| 110 | Amino acid sequence of WT IgG1 heavy chain constant region | |
| 111 | Amino acid sequence of P329G IgG1 heavy chain constant region | ASTKGPSVFPLAPSSKSTSGGTAALGCLVKDYFPEPVTVS WNSGALTSGVHTFPAVLQSSGLYSLSSVVTVPSSSLGTQ TYICNVNHKPSNTKVDKKVEPKSCDKTHTCPPCPAPELL GGPSVFLFPPKPKDTLMISRTPEVTCVVVDVSHEDPEVKF NWYVDGVEVHNAKTKPREEQYNSTYRVVSVLTVLHQD WLNGKEYKCKVSNKALGAPIEKTISKAKGQPREPQVYT LPPSRDELTKNQVSLTCLVKGFYPSDIAVEWESNGQPEN NYKTTPPVLDSDGSFFLYSKLTVDKSRWQQGNVFSCSV MHEALHNHYTQKSLSLSPGK |
| 112 | Amino acid sequence of κ light chain constant region | |

## Claims

1. An antibody that specifically binds to BCMA or antigen-binding fragment thereof, comprising
(a) three CDRs in the amino acid sequence of heavy chain variable region shown in SEQ ID NO: 27 and three CDRs in the amino acid sequence of light chain variable region shown in SEQ ID NO: 36; or variants with a single CDR or several CDRs not exceeding 2 or 1 amino acid change per CDR region within the six CDR regions;
(b) three CDRs in the amino acid sequence of heavy chain variable region shown in SEQ ID NO: 45 and three CDRs in the amino acid sequence of light chain variable region shown in SEQ ID NO: 54; or variants with a single CDR or several CDRs not exceeding 2 or 1 amino acid change per CDR region within the six CDR regions;
(c) three CDRs in the amino acid sequence of heavy chain variable region shown in SEQ ID NO: 81 and three CDRs in the amino acid sequence of light chain variable region shown in SEQ ID NO: 90; or variants with a single CDR or several CDRs not exceeding 2 or 1 amino acid change per CDR region within the six CDR regions; or
(d) three CDRs in the amino acid sequence of heavy chain variable region shown in SEQ ID NO: 99 and three CDRs in the amino acid sequence of light chain variable region shown in SEQ ID NO: 108; or variants with a single CDR or several CDRs not exceeding 2 or 1 amino acid change per CDR region within the six CDR regions;
wherein the amino acid change is the addition, deletion or substitution of amino acid.

2. An antibody that specifically binds to BCMA or antigen-binding fragment thereof, comprising a heavy chain variable region and a light chain variable region, wherein
(a) the heavy chain variable region comprises HCDR1 shown in GSIVSSSYYWT (SEQ ID NO: 19), or a variant of the HCDR1 with no more than 2 amino acid changes or no more than 1 amino acid change; HCDR2 shown in SISIAGSTYYNPSLKS (SEQ ID NO: 20), or a variant of the HCDR2 with no more than 2 amino acid changes or no more than 1 amino acid change; HCDR3 shown in ARDRGDTILDV (SEQ ID NO: 21), or a variant of the HCDR3 with no more than 2 amino acid changes or no more than 1 amino acid change, according to Kabat numbering; the light chain variable region comprises LCDR1 shown in RASQSISRYLN (SEQ ID NO: 28) , or a variant of the LCDR1 with no more than 2 amino acid changes or no more than 1 amino acid change; LCDR2 shown in AASSLQS (SEQ ID NO: 29), or a variant of the LCDR2 with no more than 2 amino acid changes or no more than 1 amino acid change; and LCDR3 shown in QQKYFDIT (SEQ ID NO: 30), or a variant of the LCDR3 with no more than 2 amino acid changes or no more than 1 amino acid change, according to Kabat numbering;
(b) the heavy chain variable region comprises HCDR1 shown in GSIVSSSYYWT (SEQ ID NO: 37), or a variant of the HCDR1 with no more than 2 amino acid changes or no more than 1 amino acid change; HCDR2 shown in SISIAGSTYYNPSLKS (SEQ ID NO: 38), or a variant of the HCDR2 with no more than 2 amino acid changes or no more than 1 amino acid change; HCDR3 shown in ARDRGDQILDV (SEQ ID NO: 39), or a variant of the HCDR3 with no more than 2 amino acid changes or no more than 1 amino acid change, according to Kabat numbering; the light chain variable region comprises LCDR1 shown in RASQSISRYLN (SEQ ID NO: 46), or a variant of the LCDR1 with no more than 2 amino acid changes or no more than 1 amino acid change; LCDR2 shown in AASSLQS (SEQ ID NO: 47), or a variant of the LCDR2 with no more than 2 amino acid changes or no more than 1 amino acid change; and LCDR3 shown in QQKYFDIT (SEQ ID NO: 48), or a variant of the LCDR3 with no more than 2 amino acid changes or no more than 1 amino acid change, according to Kabat numbering;
(c) the heavy chain variable region comprises HCDR1 shown in GTFSNDVIS(SEQ ID NO: 73), or a variant of the HCDR1 with no more than 2 amino acid changes or no more than 1 amino acid change; HCDR2 shown in VIIPIFGIANYAQKFQG(SEQ ID NO: 74), or a variant of the HCDR2 with no more than 2 amino acid changes or no more than 1 amino acid change; HCDR3 shown in ARGRGYYSSWLLDI(SEQ ID NO: 75), or a variant of the HCDR3 with no more than 2 amino acid changes or no more than 1 amino acid change, according to Kabat numbering; the light chain variable region comprises LCDR1 shown in QASQDITNYLN(SEQ ID NO: 82), or a variant of the LCDR1 with no more than 2 amino acid changes or no more than 1 amino acid change; LCDR2 shown in DASNLET(SEQ ID NO: 83), or a variant of the LCDR2 with no more than 2 amino acid changes or no more than 1 amino acid change; and LCDR3 shown in QQAFDLIT(SEQ ID NO: 84), or a variant of the LCDR3 with no more than 2 amino acid changes or no more than 1 amino acid change, according to Kabat numbering; or
(d) the heavy chain variable region comprises HCDR1 shown in GTFSNDVIS(SEQ ID NO: 91), or a variant of the HCDR1 with no more than 2 amino acid changes or no more than 1 amino acid change; HCDR2 shown in VIIPIFGIANYAQKFQG(SEQ ID NO: 92), or a variant of the HCDR2 with no more than 2 amino acid changes or no more than 1 amino acid change; HCDR3 shown in ARGRGYYSSWLHDI(SEQ ID NO: 93), or a variant of the HCDR3 with no more than 2 amino acid changes or no more than 1 amino acid change, according to Kabat numbering; the light chain variable region comprises LCDR1 shown in QASQDITNYLN(SEQ ID NO: 100), or a variant of the LCDR1 with no more than 2 amino acid changes or no more than 1 amino acid change; LCDR2 shown in DASNLET(SEQ ID NO: 101), or a variant of the LCDR2 with no more than 2 amino acid changes or no more than 1 amino acid change; and LCDR3 shown in QQAFDLIT(SEQ ID NO: 102), or a variant of the LCDR3 with no more than 2 amino acid changes or no more than 1 amino acid change, according to Kabat numbering;
wherein the amino acid change is the addition, deletion or substitution of amino acid.

3. The antibody that specifically binds to BCMA or antigen-binding fragment thereof according to claim 2, comprising a heavy chain variable region and a light chain variable region, wherein
(a) the heavy chain variable region comprises HCDR1 shown in GSIVSSSYYWT (SEQ ID NO: 19); HCDR2 shown in SISIAGSTYYNPSLKS (SEQ ID NO: 20); and HCDR3 shown in ARDRGDTILDV (SEQ ID NO: 21); the light chain variable region comprises LCDR1 shown in RASQSISRYLN (SEQ ID NO: 28); LCDR2 shown in AASSLQS (SEQ ID NO: 29); and LCDR3 shown in QQKYFDIT (SEQ ID NO: 30);
(b) the heavy chain variable region comprises HCDR1 shown in GSIVSSSYYWT (SEQ ID NO: 37); HCDR2 shown in SISIAGSTYYNPSLKS (SEQ ID NO: 38); and HCDR3 shown in ARDRGDQILDV (SEQ ID NO: 39); the light chain variable region comprises LCDR1 shown in RASQSISRYLN (SEQ ID NO: 46); LCDR2 shown in AASSLQS (SEQ ID NO: 47); and LCDR3 shown in QQKYFDIT (SEQ ID NO: 48);
(c) the heavy chain variable region comprises HCDR1 shown in GTFSNDVIS(SEQ ID NO: 73); HCDR2 shown in VIIPIFGIANYAQKFQG(SEQ ID NO: 74); HCDR3 shown in ARGRGYYSSWLLDI(SEQ ID NO: 75); the light chain variable region comprises LCDR1 shown in QASQDITNYLN(SEQ ID NO: 82); LCDR2 shown in DASNLET(SEQ ID NO: 83); and LCDR3 shown in QQAFDLIT(SEQ ID NO: 84); or
(d) the heavy chain variable region comprises HCDR1 shown in GTFSNDVIS(SEQ ID NO: 91); HCDR2 shown in VIIPIFGIANYAQKFQG(SEQ ID NO: 92); HCDR3 shown in ARGRGYYSSWLHDI(SEQ ID NO: 93); the light chain variable region comprises LCDR1 shown in QASQDITNYLN(SEQ ID NO: 100); LCDR2 shown in DASNLET(SEQ ID NO: 101); and LCDR3 shown in QQAFDLIT(SEQ ID NO: 102).

4. The antibody that specifically binds to BCMA or antigen-binding fragment thereof according to any of claims 1 to 3, comprising a heavy chain variable region and a light chain variable region, wherein
(a) the heavy chain variable region comprises a sequence shown in SEQ ID NO: 27 or a sequence having at least 90%, 91%, 92%, 93%, 94%, 95%, 96%, 97%, 98% or 99% identity thereto, and the light chain variable region comprises a sequence shown in SEQ ID NO: 36 or a sequence having at least 90%, 91%, 92%, 93%, 94%, 95%, 96%, 97%, 98% or 99% identity thereto;
(b) the heavy chain variable region comprises a sequence shown in SEQ ID NO: 45 or a sequence having at least 90%, 91%, 92%, 93%, 94%, 95%, 96%, 97%, 98% or 99% identity thereto, and the light chain variable region comprises a sequence shown in SEQ ID NO: 54 or a sequence having at least 90%, 91%, 92%, 93%, 94%, 95%, 96%, 97%, 98% or 99% identity thereto;
(c) the heavy chain variable region comprises a sequence shown in SEQ ID NO: 81 or a sequence having at least 90%, 91%, 92%, 93%, 94%, 95%, 96%, 97%, 98% or 99% identity thereto, and the light chain variable region comprises a sequence shown in SEQ ID NO: 90 or a sequence having at least 90%, 91%, 92%, 93%, 94%, 95%, 96%, 97%, 98% or 99% identity thereto;
(d) the heavy chain variable region comprises a sequence shown in SEQ ID NO: 99 or a sequence having at least 90%, 91%, 92%, 93%, 94%, 95%, 96%, 97%, 98% or 99% identity thereto, and the light chain variable region comprises a sequence shown in SEQ ID NO: 108 or a sequence having at least 90%, 91%, 92%, 93%, 94%, 95%, 96%, 97%, 98% or 99% identity thereto.

5. The antibody that specifically binds to BCMA or antigen-binding fragment thereof according to claim 4, comprising a heavy chain variable region and a light chain variable region, wherein said antibody or antigen binding fragment comprises
(a) a heavy chain variable region shown in SEQ ID NO: 27 and a light chain variable region shown in SEQ ID NO: 36;
(b) a heavy chain variable region shown in SEQ ID NO: 45 and a light chain variable region shown in SEQ ID NO: 54;
(c) a heavy chain variable region shown in SEQ ID NO: 81 and a light chain variable region shown in SEQ ID NO: 90; or
(d) a heavy chain variable region shown in SEQ ID NO: 99 and a light chain variable region shown in SEQ ID NO: 108.

6. The antibody that specifically binds to BCMA or antigen-binding fragment thereof according to any of claims 1 to 5, which is IgG1, IgG2, IgG3 or IgG4 antibody; optionally, which is IgG1 or IgG4 antibody; optionally, which is IgG1 antibody.

7. The antibody that specifically binds to BCMA or antigen-binding fragment thereof according to any of claims 1 to 6, wherein the antigen binding fragment is Fab, Fab', F(ab')2, Fv, single chain Fv, single chain Fab or diabody.

8. The antibody that specifically binds to BCMA or antigen-binding fragment thereof according to any of claims 1 to 6, further comprising a mutant Fc domain, wherein the amino acid at position P329 according to EU numbering is mutated to glycine (G), and Fcy receptor binding of the mutant Fc domain is reduced, compared with Fcy receptor binding of the Fc domain of the parent antibody that is not mutated; for example, the mutant Fc domain is a mutant Fc domain of an IgG1, IgG2, IgG3 or IgG4 antibody; preferably, the mutant Fc domain is a mutant Fc domain of an IgG1 or IgG4 antibody; more preferably, the mutant Fc domain is a mutant Fc domain of an IgG1 antibody;
For example, the antibody or antigen-binding fragment comprises a heavy chain constant region sequence shown in SEQ ID NO: 111, or a sequence having at least 90%, 91%, 92%, 93%, 94%, 95%, 96%, 97%, 98% or 99% identity thereto and having the amino acid at position P329 according to EU numbering mutated to G;
For example, the antibody or antigen-binding fragment comprises a heavy chain constant region sequence shown in SEQ ID NO: 111, or a sequence having at least 90%, 91%, 92%, 93%, 94%, 95%, 96%, 97%, 98% or 99% identity thereto and having the amino acid at position P329 according to EU numbering mutated to G; and a light chain constant region sequence shown in SEQ ID NO: 112, or a sequence having at least 90%, 91%, 92%, 93%, 94%, 95%, 96%, 97%, 98% or 99% identity thereto;
For example, the antibody or antigen-binding fragment comprises a heavy chain constant region sequence shown in SEQ ID NO: 111 and a light chain constant region sequence shown in SEQ ID NO: 112.

9. The antibody that specifically binds to BCMA or antigen-binding fragment thereof according to any of claims 1 to 8, having one or more of the following properties:
(1) binding BCMA, such as human BCMA, cynomolgus monkey BCMA and mouse BCMA, with a high affinity, for example, the binding of the anti-BCMA antibody or antigen-binding fragment thereof to BCMA has K_{D} of about 10⁻⁹M to about 10⁻¹²M, as measured by ForteBio Kinetic Binding Assay;
(2) specifically binding BCMA expressed on the cell surface;
(3) having ADCC cytotoxic killing effect on cells expressing BCMA;
(4) having ADCP killing effect on cells expressing BCMA;
(5) blocking, inhibiting the growth of cells expressing human BCMA (especially multiple myeloma cells), and/or killing said cells; and
(6) having an *in vivo* anti-tumor effect on tumors expressing BCMA, and having no significant toxic and side effects.

10. An isolated nucleic acid, encoding the anti-BCMA antibody or antigen-binding fragment thereof according to any of claims 1 to 9.

11. A vector comprising the nucleic acid of claim 10, preferably the vector is an expression vector.

12. A host cell comprising the nucleic acid of claim 10 or comprising the vector of claim 11, preferably, the host cell is prokaryotic or eukaryotic, more preferably selected from Escherichia coli cells, yeast cells, mammalian cells or other cells suitable for preparing the antibody or antigen-binding fragment thereof, and most preferably, the host cell is HEK293 cells or CHO cells.

13. A method for preparing the anti-BCMA antibody or antigen-binding fragment thereof according to any of claims 1 to 9, comprising cultivating the host cell of claim 12 under conditions suitable for expressing the nucleic acid encoding the anti-BCMA antibody or antigen-binding fragment thereof according to claims 1 to 9, optionally isolating the anti-BCMA antibody or antigen-binding fragment thereof, and optionally further comprising recovering the anti-BCMA antibody or antigen-binding fragment thereof from the host cell.

14. A conjugate, a fusion or a bispecific antibody comprising the anti-BCMA antibody or antigen-binding fragment thereof according to any of claims 1 to 9.

15. A pharmaceutical composition, comprising the anti-BCMA antibody or antigen-binding fragment thereof according to any of claims 1 to 9, or comprising the conjugate, the fusion or the bispecific antibody according to claim 14, and optionally a pharmaceutically acceptable carrier.

16. Use of the anti-BCMA antibody or antigen-binding fragment thereof according to any of claims 1 to 9, the conjugate, the fusion or the bispecific antibody according to claim 14, or the pharmaceutical composition according to claim 15 in the preparation of a medicament for preventing or treating B-cell related diseases in a subject, for example, the B-cell related diseases are selected from a group consisting of B cell malignant tumors, plasma cell malignant tumors and autoimmune diseases, preferably selected from a group consisting of multiple myeloma, non-Hodgkin's lymphoma, B cell proliferation with uncertain malignant potential, lymphomatoid granulomatosis, post-transplant lymphoproliferative diseases, immune regulatory diseases, rheumatoid arthritis, myasthenia gravis, idiopathic thrombocytopenic purpura, antiphospholipid syndrome, Chagas disease, Graves' disease, Wegener's granulomatosis, polyarteritis nodosa, Schegren syndrome, pemphigus vulgaris, scleroderma, multiple sclerosis, ANCA-associated vasculitis, Goodpasture's disease, Kawasaki disease, autoimmune hemolytic anemia, and rapidly progressive glomerulonephritis, heavy chain disease, primary or immune cell related amyloidosis, or monoclonal gammopathy of undetermined significance, preferably, the B cell related disease is a B cell malignant tumor, more preferably, multiple myeloma (MM) or non-Hodgkin's lymphoma (NHL).

17. A kit for detecting BCMA in a sample, comprising the anti-BCMA antibody or antigen-binding fragment thereof according to any of claims 1 to 9, wherein the kit is used to perform the following steps:
(a) contacting the sample with the anti-BCMA antibody or antigen-binding fragment thereof according to any of claims 1 to 9; and
(b) detecting the formation of the complex between the anti-BCMA antibody or antigen-binding fragment thereof and BCMA; optionally, the anti-BCMA antibody or antigen-binding fragment thereof is detectably labeled.
